# EUROPEAN PATENT SPECIFICATION

(11) **EP 2 962 114 B1**
(45) Date of publication and mention of the grant of the patent: **16.05.2018**
(21) Application number: 14709373.6
(22) Date of filing: 26.02.2014
(51) Int. Cl.: G01N 33/68

(54) **ASSAY FOR POLYPEPTIDE AGGREGATION USING MICRODROPLETS**
POLYPEPTIDAGGREGATIONSTEST MIT MIKROTRÖPFCHEN
DOSAGE DE L'AGRÉGATION DE POLYPEPTIDES À L'AIDE DE MICROGOUTTELETTES

(30) Priority: 27.02.2013 GB 201303507
(43) Date of publication of application: 06.01.2016
(73) Proprietor: Crowther, Damian C., Cambridge CB1 3BX (GB); Hollfelder, Florian, Cambridge CB4 3EL (GB); Gielen, Fabrice, Exeter EX4 5EW (GB); Van Vliet, Liisa, Cambridge CB4 3QU (GB); Bauer (nee YAN), Jing, 91623 Sachsen b. Ansbach (DE); Fischlechner, Martin, 6165 Telfes i. Stubai (AT); Kaminski, Clemens, Cambridge CB23 7BG (GB); Kaminski, Gabi, Cambridge CB23 7BG (GB)
(72) Inventor: Crowther, Damian C., Cambridge CB1 3BX (GB); Hollfelder, Florian, Cambridge CB4 3EL (GB); Gielen, Fabrice, Exeter EX4 5EW (GB); Van Vliet, Liisa, Cambridge CB4 3QU (GB); Bauer (nee YAN), Jing, 91623 Sachsen b. Ansbach (DE); Fischlechner, Martin, 6165 Telfes i. Stubai (AT); Kaminski, Clemens, Cambridge CB23 7BG (GB); Kaminski, Gabi, Cambridge CB23 7BG (GB)
(74) Representative: Carpmaels & Ransford LLP
(86) International application number: PCT/GB2014/050573
(87) International publication number: WO 2014/132053

(56) References cited:
- T. P. J. KNOWLES ET AL: "Observation of spatial propagation of amyloid assembly from single nuclei", PROCEEDINGS OF THE NATIONAL ACADEMY OF SCIENCES, vol. 108, no. 36, 6 September 2011 (2011-09-06), pages 14746-14751, XP055119862, ISSN: 0027-8424, DOI: 10.1073/pnas.1105555108 cited in the application
- MATTHIAS MEIER ET AL: "Plug-Based Microfluidics with Defined Surface Chemistry to Miniaturize and Control Aggregation of Amyloidogenic Peptides", ANGEWANDTE CHEMIE INTERNATIONAL EDITION, vol. 48, no. 8, 9 February 2009 (2009-02-09), pages 1487-1489, XP055119740, ISSN: 1433-7851, DOI: 10.1002/anie.200805225 & Matthias Meier ET AL: "Supporting Information for Plug-Based Microfluidics with Defined Surface Chemistry to Miniaturize and Control Aggregation of Amyloidogenic Peptides", , 1 January 2009 (2009-01-01), XP055120438, Retrieved from the Internet: URL:http://onlinelibrary.wiley.com/store/1 0.1002/anie.200805225/asset/supinfo/anie_2 00805225_sm_miscellaneous_information.pdf? v=1&s=b9b2651f291c0d8112b34f76567503cecd1d e06c [retrieved on 2014-05-27]
- S. GUPTA ET AL: "Protein Misfolding Detected Early in Pathogenesis of Transgenic Mouse Model of Huntington Disease Using Amyloid Seeding Assay", JOURNAL OF BIOLOGICAL CHEMISTRY, vol. 287, no. 13, 20 December 2011 (2011-12-20), pages 9982-9989, XP055120281, ISSN: 0021-9258, DOI: 10.1074/jbc.M111.305417

## Description

### TECHNICAL FIELD

This invention is in the field of assays for aggregatory proteins.

### BACKGROUND TO THE INVENTION

The increased propensity of proteins to misfold and aggregate in the aged organism is thought to underlie many diseases of old age such as Alzheimer's, Parkinson's, motor neurone and prion diseases. These disorders are characterised by aggregates of specific polypeptides and, in the case of Alzheimer's disease (AD), these are extracellular plaques and intracellular tangles. The plaques are of particular interest because overproduction of the constituent peptide, amyloid beta (Aβ), is sufficient to cause rare familial forms of AD.

There is significant interest in understanding the fundamental processes underlying the aggregation that causes these conformational diseases, which should assist with the development of therapeutics to slow or stop the aggregation. Given the central role that protein aggregation has in the pathology of the conformational diseases, there is a need in the art for improved methods for testing the effects of substances on aggregation, in order to identify and develop therapeutics.

The generation of biologically toxic protein aggregates is amenable to study *in vitro,* where the reaction is seen to occur spontaneously following an initial delay called the lag phase. In a process called seeding, it is also observed that the addition of a few pre-formed aggregates will greatly accelerate the formation of protein aggregates, particularly in those cases where amyloid fibrils are the end product. The presence of such aggregatory seeds has been measured in bulk samples from experimental organisms such as worms and mice and has been observed in human tissue [1-3]. Similar seeding processes are thought to be fundamental to the infectivity that is seen in prion diseases such as bovine spongiform encephalopathy and variant Creutzfeldt-Jacob diseases. Moreover, the spread of pathology within the brain of patients suffering from the common neurodegenerative disorders may occur in a similar way. Indeed, seeding may directly result in the relentless clinical progression that is so distressing to patients and their carers [4].

Current technology allows the measurement of protein aggregation in macroscopic samples using dyes such as Congo red, thioflavin T, thioflavin S and luminescent-conjugated oligothiophenes [5] that generate a fluorescence signal when they are exposed to amyloid-like protein aggregates. However, these bulk phase techniques are insufficiently sensitive for use as a diagnostic test, because background spontaneous seeding will mask the specific effects of seeding and any specific signal is diluted into the bulk of a typical test solution. A highly sensitive and specific test will be required if aggregatory seeds, at low concentrations in peripheral tissues and fluids such as blood and urine, are to be detected [1]. Current diagnostic tests for conformational diseases caused by the aggregation of proteins, such as Alzheimer's disease, typically rely on algorithms based on neuropsychological tests and brain imaging, in part because the protein that aggregates is present in healthy individuals, so the utility of these proteins as biomarkers is limited. There is therefore a need in the art for new and improved diagnostic assays that can accurately detect the presence of clinically relevant species representing a particular conformation of a protein or an aggregate of polypeptides with a particular conformation. Improved assays are also required for screening candidate therapeutics for an ability to modulate clinically relevant processes underlying the aggregation of proteins that cause conformational diseases.
Knowles et al. (2011, PNAS, 108(36): 14746-14751) describes an assay for observing spatial propagation of amyloid assembly from single nuclei.
Meier et al. (2009, Angew Chem Int Ed Engl., 48(8): 1487-1489) describes plug-based microfluidics with defined surface chemistry intended to miniaturise and control aggregation of amyloidogenic peptides.
Gupta et al. (2011, The Journal of Biological Chemistry, 287(13): 9982-9989) describes an assay for detecting misfolded huntingtin protein.

### SUMMARY OF THE INVENTION

The invention provides a method for detecting the presence of aggregatory seeds of a polypeptide in a sample, wherein an aggregatory seed is a soluble oligomer of the polypeptide, comprising:
(i) contacting the sample with monomers of the polypeptide, wherein the monomers of the polypeptide are labelled;
(ii) separating the sample into a plurality of microdroplets comprising a gel-forming agent in liquid form;
(iii) incubating the microdroplets under conditions suitable for aggregation of the polypeptide;
(iv) transforming the microdroplets into gel beads;
(v) determining the subsequent formation of aggregates of the polypeptide in the gel beads by detecting the label,
wherein the formation of aggregates of the polypeptide in a gel bead indicates the presence of one or more aggregatory seeds in the sample.

Aggregatory seeds of a polypeptide are difficult to detect specifically and sensitively when at low concentrations. This is because they are generally detected though aggregation seeding assays whereby samples comprising aggregatory seeds are contacted with monomers of the relevant polypeptide and the formation of polypeptide aggregates seeded by aggregatory seeds is detected. However, when the aggregatory seeds are present at a low concentration, aggregation seeded by aggregatory seeds is swamped by spontaneous aggregation of the polypeptide monomers and it is difficult specifically and sensitively to identify the presence of aggregates seeded by aggregatory seeds above this background spontaneous aggregation. Spontaneous aggregation is relatively slow and spatially homogeneous within the bulk phase solution. In the bulk phase the homogeneous, low level generation of signal cannot be differentiated by the spatially localised, high efficiency generation of signal. This is because in the bulk phase the locally generated signal diffuses into the bulk and so becomes indistinguishable from spontaneously generated signal.

The method of the invention is able to specifically detect seeded aggregation and to distinguish it from background spontaneous aggregation by contacting a sample with monomers of the relevant polypeptide and compartmentalising the sample into a plurality of microfluidic droplets. By separating a sample into a plurality of microdroplets, the local concentration of any aggregatory seeds is increased to the extent that seeded aggregation in microdroplets comprising aggregatory seeds is specifically and sensitively detectable above background spontaneous aggregation.

Therefore, compartmentalising the sample into microdroplets is particularly advantageous for detecting aggregatory seeds using an aggregation assay, because the monomers of the polypeptide that are required for the aggregation assay are liable to spontaneously aggregate, which produces background signal or noise, making the specific detection of aggregates resulting from aggregatory seeds difficult. By analysing a plurality of microdroplets, each of which can be positively determined to have contained or to not have contained aggregatory seeds, the invention allows a sensitive and quantitative analysis of the presence of aggregatory seeds in the initial sample.

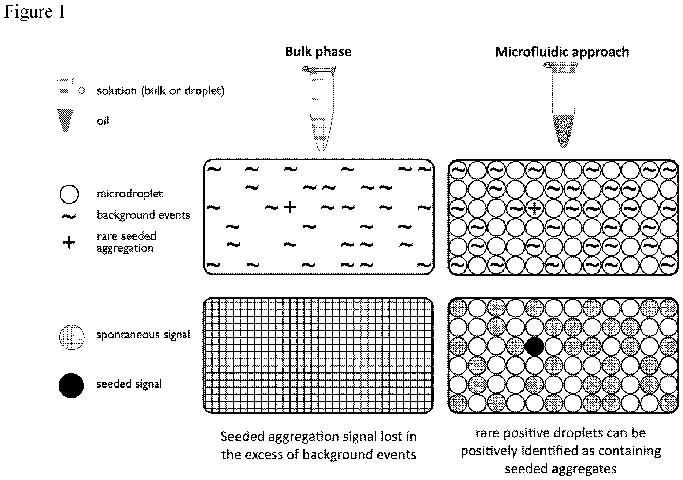

Classical biochemical experiments performed in bulk phase are not sensitive or specific enough to usefully report the presence of aggregatory seeds of a protein in a sample. To overcome the insufficiently sensitive detection limits of current methods, the inventors have developed a method that uses compartmentalisation in micro fluidic droplets, such as water-in-oil droplets, with µm diameters and picolitre volumes. The fragmentation of a bulk phase into many smaller reactors (for example, about 10⁷/ml) creates higher local concentrations. For example, if only one seed is present in a sample, the effective concentration in one of 10⁷ droplet compartments will be increased 10⁷-fold. The principle underlying the invention is demonstrated schematically in Figure 1.

The method of the invention is particularly useful in the diagnosis and monitoring of disease, because the assay can be used to detect the presence of aggregatory seeds of a polypeptide in patient samples. Therefore, the invention also provides a method for identifying a subject at risk of a conformational disease caused by the aggregation of a polypeptide or for diagnosing, assessing or monitoring a conformational disease caused by the aggregation of a polypeptide in a subject, comprising detecting the presence of aggregatory seeds of the polypeptide in a sample obtained from the subject, wherein an aggregatory seed is a soluble oligomer of the polypeptide, wherein the detecting comprises:
(i) contacting the sample with monomers of the polypeptide wherein the monomers of the polypeptide are labelled;
(ii) separating the sample into a plurality of microdroplets comprising a gel-forming agent in liquid form;
(iii) incubating the microdroplets under conditions suitable for aggregation of the polypeptide;
(iv) transforming the microdroplets into gel beads;
(v) determining the subsequent formation of aggregates of the polypeptide in the gel beads by detecting the label,
wherein the formation of aggregates of the polypeptide in a microdroplet indicates the presence of one or more aggregatory seeds in the sample, and the presence of aggregatory seeds in the sample is correlated with the risk, presence, or severity of disease.

The inventors have developed a method for sensitively and specifically detecting the presence of aggregatory seeds at very low concentrations. Therefore, the present invention makes it possible to detect the presence of aggregatory seeds in samples obtained from human subjects and to correlate the presence of aggregatory seeds with the risk, presence, or severity of a conformational disease. As noted above, by analysing a plurality of microdroplets, each of which can be positively determined to have contained or to not have contained aggregatory seeds, the invention allows a sensitive and quantitative analysis of the presence of aggregatory seeds in the initial sample, and this enables a useful diagnosis or prognosis to be made.

The method of the invention is particularly advantageous, because it enables a new class of biomarker to be analysed in the diagnosis of conformational diseases. Conformational diseases are often caused by endogenous proteins that are present in healthy individuals, which limits the utility of simply measuring the concentration of these proteins as biomarkers of disease. This is because it is the conformation of the protein or the self-association of several copies of the polypeptides in particular conformations that generates the toxic species. By sensitively and specifically detecting aggregatory seeds, the present invention allows the detection of a clinically relevant pathological form of the protein underlying the conformational disease. In addition, by detecting aggregatory seeds, the method of the invention allows earlier diagnosis of conformational disease, before large aggregates have been formed. Also, the invention represents a significant advancement because it involves quantifying an activity (seeding of aggregation), rather than merely reporting the presence of a structural epitope in a sample. This seeding activity is of fundamental importance in disease progression, so such measurements have diagnostic and prognostic value in the clinic.

Furthermore, the proteins underlying conformational diseases generally aggregate in the brain, which is inaccessible for simple diagnostic tests. By sensitively and specifically detecting aggregatory seeds, the present invention allows the detection of a clinically relevant pathological form of the protein in peripheral fluids, such as blood and urine, which is practical for diagnostic testing. Aggregatory seeds of proteins underlying conformational diseases are present in peripheral fluids, whilst larger aggregates, which might be assayed more easily, are only present in less accessible tissues, such as the brain.

Significantly, the sensitivity of the method of the invention makes it possible to detect aggregatory seeds at the very low concentrations that they are found in, in peripheral tissues such as blood and urine. As explained above, through contacting a sample with monomers of the relevant polypeptide, compartmentalising the sample into a plurality of microfluidic droplets and performing an aggregation assay to detect the formation of polypeptide aggregates, the method of the invention allows the specific and sensitive detection of aggregatory seeds at low concentrations.

In addition to methods of diagnosis, the assay of the invention will be useful for preparing reagents or products that are free from aggregatory seeds and for quantifying the amount of aggregatory seeds produced in a test reaction. For example, the assay of the invention will be useful for assessing the ability of a reagent to effect the formation of aggregatory seeds.

In certain embodiments, the microdroplets comprise droplets in an emulsion, such as a water-in-oil emulsion, and the method of the invention comprises forming an emulsion, such as a water-in-oil emulsion. Emulsions can be generated easily using standard equipment and techniques. Microdroplets can be generated in microfluidic devices, are easy to handle with microfluidic devices and they are particularly amenable to the high throughput screening, which is useful for analysing a large number of aliquots, as in the present invention. There are also a number of methods for producing bulk emulsions including vigorous stirring, vortexing, shaking and extrusion, although the resulting droplets are polydisperse.

In the invention, the microdroplet comprises a gel-forming agent and the microdroplet is transformed into a gel bead. Preferably, the microdroplets are transformed into gel beads after incubating the microdroplets under conditions suitable for aggregation of the polypeptide. By transforming the microdroplet into a gel bead, polypeptide aggregates are retained in the bead despite thorough washing. This has the advantage of allowing very sensitive and specific detection of polypeptide aggregates, because un-aggregated monomers can be washed away. Furthermore, the gel beads are stable and resilient and can be transferred between different reaction conditions, allowing further detailed analysis of the polypeptide aggregates, whilst still maintaining the compartmentalisation of the initial subject sample. Therefore, in certain embodiments, the microdroplet is transformed into a gel bead and polypeptide monomers are removed from the gel bead by washing. In certain embodiments, the gel beads are subjected to a plurality of different assays.

By performing the assay in microdroplets comprising a gel-forming agent and transforming the microdroplets into gel beads, polypeptide aggregates that are formed are immobilised in the gel beads. The assay of the invention is particularly sensitive because the gel beads can be washed extensively to remove, for example, polypeptide monomers and other reactants, and polypeptide aggregates will be retained in the gel beads. This decreases background signal from, for example, unaggregated material. The method of the invention is particularly amenable to high-throughput screening, because the gel beads are stable, resilient and easy to handle. Therefore, the assay of the invention allows large numbers of gel beads to be analysed. By accommodating the analysis of a large number of gel beads, the methods of the invention provide increased sensitivity, because detecting very rare events is possible. In addition, the ability to test a large number of beads rapidly allows many compounds to be tested. Furthermore, the resilience of the gel beads means that they can be washed and subjected to different conditions without the aggregates being lost and whilst still maintaining the compartmentalisation of the initial subject sample, which allows multiple assays to be performed.

The porous structure of the bead matrix serves to retain aggregated protein, whilst small molecule components and buffer are free to enter or exit the bead. The beads thus become a 'time capsule' for aggregated product, which can be subjected to a number of downstream analysis methods. The ability to wash resulting gel beads and remove small oligomers and molecules enhances the sensitivity of, for example, fluorescence-based assays, because the relative signal from trapped protein aggregates increases while background from un-aggregated material is removed. The small droplet volumes (for example, 47.7 pL) provide for economical experiments and multiple redundancy. For example, a total reaction volume of 1 µL can generate up to 10⁵ identical droplets and beads, which constitutes an enormous pool for either averaging techniques or individual analysis.

Therefore, the invention provides a novel assay for the study of protein self-assembly reactions where emerging protein aggregates are confined in microbeads made up of a gel 'cage'. The beads have unique properties that permit aggregation reactions to proceed at near physiological concentrations. The 'cage' is formed by co-compartmentalising liquid gel with the reagent containing solute (e.g. an aqueous solution containing peptide and drug etc.) during the droplet formation step. The droplets can be formed in an oil phase at elevated temperatures in liquid form and stored as an emulsion for the aggregation reactions to proceed. The emulsion can be cooled on ice, leading to solidification of the beads before or after the process to be studied is completed. The oil phase is then removed and the beads are washed. The porous internal structure of the gel matrix (such as an agarose polymer matrix) prevents large aggregates from escaping the beads, whilst soluble peptide, small molecules or solvent are readily removed. The retained aggregates can be readily analysed e.g. by fluorescence or other methods in a highly repeatable and quantitative fashion. The technology affords great flexibility in handling, manipulating and analysing the self-assembly of neurotoxic proteins: reagents can be added or removed, concentrations varied, and ensuing aggregates analysed optically informing on the kinetics of aggregation and the morphology of ensuing amyloid structures. The invention provides the first demonstration of this technology and shows that the aggregation of proteins, such as mutants of Aβ, can be studied in a quantitative manner at near physiological concentrations. The method of the invention can be used to apply several optical techniques to analyse the retained aggregates, including fluorescence lifetime imaging and optical super- resolution imaging, giving direct insight on the morphology of forming aggregates. In addition, the process of aggregate formation can monitored over time by quantification of the fluorescence of the retained aggregate (by measuring intrinsic fluorescence, the fluorescence of the products of seeded co-aggregation with fluorescent monomers or by measurements of fluorescence after staining of the emerging aggregate with amyloid dyes such as e.g. thioflavin T and S, Congo Red), e.g. by flow cytometric (FACS) analysis or imaging techniques. The technology is particularly useful for the rapid screening of agents which may act as modifiers of aggregation.

### DETAILED DISCLOSURE OF THE INVENTION

### Aggregatory seeds

The term "aggregatory seed" as used herein refers to a soluble oligomer of a protein or polypeptide. Typically, an oligomer comprises monomers associated through non-covalent interactions. Aggregatory seeds shorten the nucleation-associated lag phase in the kinetics of aggregate formation. Therefore, an aggregatory seed initiates and accelerates the aggregation of similar or identical monomers. For example, aggregatory seeds accelerate the fibrilisation of monomeric protein with sequence homology. Aggregatory seeds may also act via corruptive molecular templating.

Generally, an aggregatory seed comprises a single peptide species and it seeds aggregation of monomers of the same peptide species. Generally, where a polypeptide has a native structure, an aggregatory seed comprises peptides that are at least partially misfolded, but not completely denatured. Therefore, in certain embodiments, an aggregatory seed is a soluble oligomer of misfolded peptides. In certain embodiments, an aggregatory seed comprises 2-20 monomers.

### Microdroplets

A "microdroplet" is small droplet of a sample that can be manipulated using standard microfluidic technology and devices. In certain embodiments of the invention, a microdroplet has a volume of less than 1 µl, less than 500 nl, less than 100 nl, less than 10 nl, less than 1nl, less than 500 pl, less than 400 pl, less than 300 pl, less than 200 pl, less than 100 pl, or less than 50 pl. In certain embodiments of the invention, a microdroplet has a volume of between 0.5 pl and 1 µl, between 0.5 pl and 500 nl, between 1 pl and 200 nl, between 20 pl and 100 nl, between 20 pl and 1nl, between 20 pl and 500 pl, between 20 pl and 300 pl, between 20 pl and 200 pl, between 20 pl and 100 pl, between 30 pl and 100pl, or between 30 pl and 80 pl. In certain embodiments, a microdroplet has a diameter of less than 1 mm, less than 500 µm, less than 400 µm, less than 300 µm, less than 200 µm, less than 100 µm, less than 80 µm, less than 70 µm, less than 60 µm, or less than 50 µm. In certain embodiments, a microdroplet has a diameter of between 1 µm and 1mm, between 10 µm and 500 µm, between 10 µm and 250 µm, or between 20 µm and 100 µm. When used in the detection of aggregatory seeds, the size of the microdroplet allows seeded aggregation to proceed faster than spontaneous aggregation. This may also depend on the concentration of the monomers of the protein. The skilled person is able to determine the appropriate size of microdroplet and the skilled person is able to determine an appropriate concentration of the monomers of the protein.

The number of microdroplets that a sample is compartmentalised into will depend on the volume of the sample and the volume of the microdroplets. It is not necessary that the entirety of a sample of interest, for example a clinical sample, is tested and compartmentalised into microdroplets. The portion of the sample that is tested must be sufficient to provide useful information regarding the whole sample. The skilled person is able to determine the appropriate portion of sample to test. In certain embodiments, a "plurality of microdroplets" is at least 10³, 10⁴, 10⁵, 10⁶, 10⁷, 10⁸ or 10⁹ microdroplets.

Microdroplets may be produced by emulsifying the aqueous reaction mixture in a non-aqueous liquid, for example oil, comprising a surfactant. Any suitable surfactant may be used. Techniques for the production of micro fluidic aqueous droplets are well known in the art [6-12].

Microdroplets are preferably monodisperse i.e. they preferably have a uniform size.

Classically, assays for protein aggregation are carried out in microwell plates [13], the standard format for pharmaceutical and biochemical experimentation. However, recently assays have been reported that have been scaled down to nano- or even pico-litre volumes [14,15]. This miniaturization is achieved by compartmentalisation of assay components in water-in-oil emulsion droplets generated within micro fluidic devices [16-19]. The devices are capable of generating and manipulating droplets at rates of up to 10 kHz. Fully automated modular workflows can be designed [20] to incorporate physical unit operations such as droplet sorting [21-23] and fusion [24,25], and even biochemical processes can be incorporated, such as PCR [26,27], *in vitro* protein expression [28], enzymatic reactions [25,29] or cell-based assays [30-32]. The minute droplet volume between 0.5 pL and 4 nL also offers advantages for solution-based compounds screening. These attributes, coupled with advanced analytical techniques [33-37], permit droplet assays to be performed in high-throughput fashion. In addition to saving reagents, the compartmentalisation afforded by the droplet boundary enables one to design experiments and access information that is masked in conventional ensemble experiments. Furthermore, effects arising from cell-like confinement or interfacial phenomena can be mimicked inside droplet compartments.

Microdroplets may be prepared and handled using any appropriate means. For example, microfluidic devices constructed using conventional soft lithographic techniques, FF-chips or commercial glass chips may be used.

### Polypeptides prone to aggregation

The methods of the invention are suitable for use with any polypeptide or protein that is prone to aggregation. In certain embodiments, the protein is Amyloid β peptide, such as Aβ₁₋₄₀ or Aβ₁₋₄₂, tau protein, prion protein, α-Synuclein, TDP-43, FUS, huntingtin, a serpin, transthyretin, Notch 3, glial fibrillary acidic protein, seipin, islet amyloid polypeptide (IAPP), pro-IAPP, apolipoprotein AI, All or AIV, gelsolin, β2 microglobulin, rhodopsin, keratin, keratoepithelin, tubulin, surfactant protein C, odontogenic ameloblast-associated protein, calcitonin, atrial natriuretic factor, serum amyloid A, medin, or lysozyme.

In preferred embodiments, the protein is a protein that forms amyloid aggregates, such as Amyloid β peptide, Aβ₁₋₄₀, Aβ₁₋₄₂, IAPP, α-Synuclein, prior protein, or huntingtin. The seeding of aggregation by aggregatory seeds is particularly important in the formation of amyloid aggregates. In preferred embodiments, the protein is a protein that causes a neurodegenerative disorder. The detection of aggregatory seeds that cause neurodegenerative disorders is particularly important, because the aggregatory protein accumulates in the brain, so it is useful to detect aggregatory seeds in peripheral tissues. In preferred embodiments, the protein is a prion protein. In particularly preferred embodiments, the protein is an Amyloid β peptide, such as Aβ₁₋₄₀ or Aβ₁₋₄₂. Amyloid beta is a peptide of 36-43 amino acids that is processed from the Amyloid precursor protein by secretases. Aβ₁₋₄₀ is the predominant form found circulating in plasma, cerebrospinal fluid and brain interstitial fluid. Aβ₁₋₄₂ is more aggregatory and is associated with the development of disease, and with more severe disease. Both Aβ₁₋₄₀ and Aβ₁₋₄₂ are found within plaques.

### Detection and analysis of aggregates

The use of microdroplets and gel beads in the methods of the invention allows rapid, high throughput analysis of aggregates that are formed, which is useful for detecting the aggregatory seeds in a sample and for measuring the effect of a test substance on the aggregation of a protein.

The method of the invention comprises determining the subsequent formation of aggregates of the polypeptide, which comprises determining the presence of aggregates of the polypeptide in a microdroplet. In the method of the invention, the protein monomers are labelled and determining the presence of aggregates of the protein comprises detecting the label. In certain embodiments, the protein monomers are fluorescently labelled and determining the presence of aggregates of the protein comprises detecting a fluorescent signal. In preferred embodiments, the fluorescent signal is detected using a flow cytometer or a fluorescence activated cell sorter (FACS). In certain embodiments, the magnitude of the fluorescent signal is correlated with the presence of protein aggregates. In certain embodiments, the profile of the fluorescent signal obtained using FACS indicates the presence of protein aggregates. In certain embodiments, the profile of the fluorescent signal indicates the structure of the protein aggregates formed. For example, Examples 2 and 3 demonstrate that aggregates formed through seeded aggregation initiated by an aggregatory seed result in a profile that can be distinguished from aggregates formed through spontaneous aggregation. Therefore, in certain embodiments, the step of determining the presence of aggregates of the protein comprises correlating the magnitude of the fluorescent signal with the amount of protein aggregate present. In certain embodiments, the magnitude of the fluorescent signal indicates the presence of aggregatory seeds in the test sample.

As shown in Examples 2 and 3, use of fluorescently labelled protein monomers and detection of fluorescent signals allows specific detection of synthetic aggregatory seeds and aggregatory seeds derived from animal models of disease. There is a significant and readily detectable difference in the fluorescence signal generated by free protein monomers, spontaneously formed aggregates, and seeded aggregates. By incubating aliquots of seed-containing samples with solutions containing fluorescently labelled monomeric Aβ peptides in picolitre volumes, the fluorescence signal from aggregates generated from the process of seeded aggregation of the fluorescently labelled monomeric Aβ peptides was specifically measured.

As shown in Examples 7 and 8, use of fluorescently labelled protein monomers and detection of fluorescent signals allows highly sensitive measurement and monitoring of the aggregation of proteins and modulation of this aggregation.

Optical signals from fluorescently labelled protein monomers are amenable to investigation by a number of biophysical techniques. For example, in certain embodiments, fluorescence lifetime microscopy is used to detect and/or to inspect the aggregates formed. A decrease in fluorescence lifetime is observed when protein monomers aggregate into amyloid structures [37]. In addition, the fluorescence lifetime of different types of aggregates can be distinguished and a longer fluorescence lifetime is correlated with the presence of more disordered aggregates. In a method of diagnosis, more disordered aggregates and/or the presence of seeds that produce more disordered aggregates may be correlated with increased severity or risk of disease. Also, the effect of a test substance on aggregation of a protein may be detected by an alteration in the degree of order in an aggregate as detected, for example, by fluorescence lifetime microscopy.

In certain embodiments, the method used to measure the fluorescence lifetime from reporter labels may include time domain methods such as time gated detection [38], time correlated single photon counting [39], or frequency domain lifetime imaging methods [40].

In certain embodiments, protein aggregation is monitored using fluorescence anisotropy imaging microscopy (FAIM), which monitors changes in the polarisation state of emitted light which occur upon assembly of labelled monomers into aggregated species [41].

The skilled person is aware of techniques that can be used to detect the presence of aggregates in the microdroplets or gel beads in accordance with the invention, and to determine the structure of the aggregates. For example, in certain embodiments a flow cytometer, fluorescence lifetime microscopy, direct stochastic optical reconstruction microscopy (dSTORM), structured illumination microscopy or FRET microscopy is used to detect aggregates or determine the structure of aggregates. In alternative embodiments, circular dichroism is used [42,43].

The use of fluorescently labelled protein monomers allows highly sensitive detection of aggregatory seeds. While fluorescence intensity reports on the amount of aggregate produced, lifetime measurements inform on the type of aggregate formed, such as the size or degree of order, and thus provides a second way to determine whether a droplet contains seeded or un-seeded aggregates. The profile of the fluorescent signal can be used to determine further details regarding the structure of the aggregates. As shown in Example 4, fluorescence lifetime microscopy can be used to distinguish between diseased mouse brain extracts and control mouse brain extracts (which produce a signal identical to beads incubated with buffer alone), based on the presence of aggregatory seeds.

In certain embodiments, the protein monomers are labelled with an Alexa Fluor™ dye, such as Alexa647. Alexa Fluor dyes are synthesized through sulfonation of coumarin, rhodamine, xanthene (such as fluorescein), and cyanine dyes. In certain embodiments, the protein monomers are labelled with a HiLyte FluorTM dye, such as Hilyte 488. Further suitable fluorescence reporters include fluorescein and fluorescein derivatives such as O-methyl-fluorescein or fluorescein isothiocyanate (FITC), phycoerythrin, Europium, TruRed, Allophycocyanin (APC), PerCP, Lissamine, Rhodamine, B X-Rhodamine, TRITC, BODIPY-FL, FluorX, Red 613, R-Phycoerythrin (PE), NBD, Lucifer yellow, Cascade Blue, Methoxycoumarin, Aminocoumarin, Texas Red, Hydroxycoumarin, Alexa FluorTM dyes (Molecular Probes) such as Alexa FluorTM 350, Alexa FluorTM 488, Alexa FluorTM 546, Alexa FluorTM 568, Alexa FluorTM 633, Alexa FluorTM 647, Alexa FluorTM 660 and Alexa FluorTM 700, sulfonate cyanine dyes (AP Biotech), such as Cy2, Cy3, Cy3.5, Cy5, Cy5.5 and Cy7, IRD41 IRD700 (Li-Cor, Inc.), NIR-1 (Dejindom, Japan), La Jolla Blue (Diatron), DyLight™ 405, 488, 549, 633, 649, 680 and 800 Reactive Dyes (Pierce/Thermo Fisher Scientific Inc) or LI-COR™ dyes, such as IRDye™ (LI-COR™ Biosciences). In certain embodiments, the protein is a recombinant protein labelled with a fusion protein such as GFP, YFP, mRFP or TFP. The skilled person is familiar with other fusion proteins that may be used to label the protein.

In certain embodiments, the formation of aggregates is detected using a reporter substance. For example, in certain embodiments, the sample and the monomers of protein are mixed with a reporter substance and the step of detecting the formation of aggregates of the protein comprises detecting the reporter substance. In such embodiments, the reporter substance generates a specific signal when in the presence of protein aggregates, such as amyloid aggregates. In certain embodiments, the reporter substance is Thioflavin T, Congo red, or (trans,trans)-1-bromo-2,5-bis-(3-hydroxycarbonyl-4-hydroxy)styrylbenzene (BSB).

In certain embodiments of the invention, certain beads or droplets are isolated based on the degree or structure of aggregates formed. The isolated beads or droplets can then be analysed further. This is particularly useful when gel beads are used, because the beads retain protein aggregates and enable buffers etc. to be exchanged for further analytical reactions to be performed. For example, in certain embodiments, the method of the invention comprises isolating beads or droplets comprising aggregates formed by seeded aggregation, or aggregates of a certain size.

Furthermore, in certain embodiments of the invention, the structure of the aggregates formed during the assay are analysed to provide a more detailed diagnosis. Therefore, in certain embodiments, the step of determining the presence of aggregates of the protein comprises determining the structure or one or more biophysical characteristics of the aggregates. Alternatively, in certain embodiments, the method comprises an additional step of determining the structure or one or more biophysical characteristics of the aggregates. In such embodiments, the structure and biophysical characteristics of the aggregates are used to classify the aggregatory seeds present in the sample obtained from the subject, which is used to provide a diagnosis. For example, in certain embodiments, the presence of aggregatory seeds that seed more disordered aggregates is correlated with increased severity or risk of disease. In certain embodiments, the presence of aggregatory seeds that seed more rapid aggregation of protein is correlated with increased severity or risk of disease.

Therefore, in certain embodiments of the invention, the step of determining the presence of aggregates of the protein in the microdroplets comprises detecting the presence or absence of aggregates of the protein; measuring or quantifying the amount of aggregates of the protein; and/or analysing the structure (for example, the degree of order) of the aggregates of the protein.

In certain embodiments of the invention, the sample to be tested is diluted prior to use in the assay. For example, the sample, such as a clinical sample obtained from a subject, may be diluted 5, 10, 50, 100 or 1000 times. The sensitivity and specificity of the assay and its ability to detect the presence of aggregatory seeds may be improved through such dilution.

The steps of any method disclosed herein may be performed in any order.

Usually the raw data obtained from an assay require some sort of manipulation prior to their use. For instance, the nature of most detection techniques means that some signal will sometimes be seen even if no aggregatory seed or aggregate is actually present and so this noise may be removed before the results are interpreted. Similarly, there may be a background level of the aggregatory seed in the general population which needs to be compensated for. Background signal may comprise a second peak due to background or a possible background rate in creating the second peak.

Data may need scaling or standardising to facilitate inter-experiments comparisons. These and similar issues, and techniques for dealing with them, are well known in the diagnostic and fluorescence microscopy areas.

Various techniques are available to compensate for background signal in a particular experiment. For example, replicate measurements will usually be performed to determine intra-assay variation, and average values from the replicates can be compared. Signal may be adjusted according to distribution in a single experiment. Data transformations of this type are standard in the art for permitting valid inter-array comparisons despite variation between different experiments.

### Methods of diagnosing, assessing, monitoring or identifying risk for disease

As discussed above, the methods of the invention are particularly useful for detecting the presence of aggregatory seeds in a patient sample in order to provide a diagnosis, prognosis or to identify risk for developing a disease.

The method of the invention is useful for diagnosis, prognosis and diagnosis of risk for diseases caused by the aggregation of a particular protein. Such diseases are known as conformational diseases.

As will be clear to the skilled person, the method of the invention is used to diagnose, assesses, monitor or identify risk for the conformational disease that is caused by aggregation of the protein of which aggregatory seeds are detected. Therefore, aggregatory seeds of Aβ₁₋₄₀ or Aβ₁₋₄₂ are detected in the diagnosis of Alzheimer's disease, and aggregatory seeds of α-synuclein are detected in the diagnosis of Parkinson's disease, etc. The method of the invention is useful for diagnosing, assessing, monitoring and identifying risk for Alzheimer's disease, Parkinson's disease and other synucleinopathies, tauopathies, prion diseases, cerebral β-amyloid angiopathy, CADASIL syndrome, frontotemporal lobar degeneration, FTLD-FUS, amyotrophic lateral sclerosis, Huntington's disease and other triplet repeat disorders, hereditary cerebral hemorrhage with amyloidosis, Alexander disease, seipinopathies, familial amyloidotic neuropathy, senile systemic amyloidosis, serpinopathies, type II diabetes, aortic medial amyloidosis, ApoAI, AII or AIV amyloidosis, familial amyloidosis of the Finnish type, lysozyme amyloidosis, fibrinogen amyloidosis, dialysis amyloidosis, inclusion body myositis/myopathy, retinitis pigmentosa with rhodopsin mutations, medullary thyroid carcinoma, cardiac atrial amyloidosis, corneal dystrophy, cutaneous lichen amyloidosis, pulmonary alveolar proteinosis, odontogenic (Pindborg) tumor, atherosclerosis, and systemic amyloidoses such as hereditary non-neuropathic systemic amyloidosis, AA and AL.

The central role that aggregatory seeds play in the pathogenesis of conformational disease is well established. Seeded (prion-like) protein aggregation is understood to spread out from an initial nidus [4]. Murine models of seeded aggregation have implicated both Aβ and also the intracellular protein tau in the pathology of Alzheimer's disease [2,3,44-46]. For both Aβ and tau it has been shown that inoculation of model rodents with preformed aggregates can accelerate the pathological deposition of plaques and tangles, something that would eventually occur spontaneously in these rodents. Furthermore, there is good evidence that similar inoculation can seed novel tau pathology in rodents that would otherwise remain healthy [2,46]. The seeded aggregation of proteins is particularly well understood in relation to prion proteins and the formation of amyloid. The prion protein can misfold and aggregate, either stochastically or as the result of a mutation, and the resulting seeds are able to spread within, and between, individuals creating a cascade of misfolding and neurotoxicity [47,48]. *In vitro* studies of aggregation and amyloid formation have shown that a pure solution of monomers is often stable for an extended period of time called the lag phase. During this time a stochastic process of seed formation occurs, resulting in a burst of amyloid formation as fibrils grow and fracture and so generate more sites for the rapid addition of monomeric peptide [49,50]. These fibrils, when doped back into a subsequent aggregation reaction will often abolish the lag phase, accelerating the formation of further amyloid. Prion-like infectivity through aggregatory seeds has also been demonstrated in other conformational diseases, such as Huntington's disease [1], Parkinson's disease and motor neurone disease [51]. Therefore, the detection of aggregatory seeds is likely to be useful and effective in the diagnosis of conformational diseases.

In preferred embodiments, the method of the invention is a method for diagnosing, assessing, monitoring or identifying risk for a neurodegenerative disease. The methods of the invention are particularly useful for neurodegenerative diseases, because while the brain is not accessible, nevertheless aggregatory seeds can be found in peripheral fluids, such as blood.

In preferred embodiments, the method of the invention is a method for diagnosing, assessing, monitoring or identifying risk for an amyloidopathy. The methods of the invention are particularly useful for amyloidopathies, because the seeding of aggregation by aggregatory seeds is particularly important in the formation of amyloid aggregates, so a diagnosis that includes detection of aggregatory seeds is likely to be particularly accurate and useful.

In particularly preferred embodiments, the method of the invention is a method for identifying a subject at risk of Alzheimer's disease or for diagnosing, assessing or monitoring Alzheimer's disease.

The subject will typically be a human being. In some embodiments, however, the invention is useful in non-human organisms e.g. horse, pig, cow, sheep, deer, chicken, mouse, rat, rabbit, guinea pig, cat, dog,, or non-human primate (monkeys or apes, such as macaques or chimpanzees).

In some embodiments, a method of the invention involves an initial step of obtaining the sample from the subject. In other embodiments, however, the sample is obtained separately from and prior to performing a method of the invention. After a sample has been obtained then methods of the invention could be performed *in vitro.*

In certain diagnostic methods of the invention, the sample tested is a blood sample (such as a serum or plasma sample), a urine sample, a cerebrospinal fluid sample, a saliva sample, a tears sample, a lymphatic fluid sample or a urine sample. As discussed above, the sensitive methods of the invention are suitable for detecting aggregatory seeds at low concentrations in peripheral fluids. In preferred embodiments of the invention, the sample is a serum or plasma sample.

Detection of aggregatory seeds may be performed directly on a sample taken from a subject, or the sample may be treated between being taken from a subject and being analysed. For example, a blood sample may be treated to remove cells, leaving plasma containing aggregatory seeds for analysis, or to remove cells and various clotting factors, leaving serum potentially containing aggregatory seeds for analysis. For some body fluids, such separation treatments are not usually required (e.g. urine, tears or saliva) but other treatments may be used. For example, various types of sample may be subjected to treatments such as dilution, aliquoting, sub-sampling, heating, freezing, irradiation, etc. between being taken from the body and being analysed e.g. serum is usually stored, frozen prior to analysis. Also, addition of processing reagents is typical for various sample types e.g. addition of anticoagulants to blood samples.

In certain embodiments of the invention, the method additionally comprises performing additional tests and analyses. For example, a diagnosis will generally also include consideration of the subject's age, their family history of disease, other biomarkers, and/or any symptoms. In addition, assessing a subjects risk for a disease will generally include numerous variables including the subject's age, their family history of disease, other biomarkers, and lifestyle. An algorithm may be used to arrive at an assessment of disease risk. The skilled person is aware of how to develop a suitable algorithm.

In certain embodiments of the invention, the presence or levels of aggregatory seeds in a patient's clinical sample may be compared to a control sample. The control sample may be derived from a patient or group of patients that do not have disease, or a patient or group of patients that do have disease. The control sample may be a blank sample. In certain embodiments of the invention, the presence or levels of aggregatory seeds in a patient's sample may be compared to a reference value. In certain embodiments, the presence or levels of aggregatory seeds in a patient's clinical sample may be compared to a threshold value, wherein the presence or levels of aggregatory seeds over the threshold value indicates the presence of the disease condition, or indicates increased risk for the disease condition. The reference or threshold value may be derived from analysis of a large number of patient samples. This analysis may comprise comparing samples from healthy patients and diseased patients. This analysis may be a longitudinal study comparing samples from patients before and after developing disease.

The methods of the invention may comprise monitoring a patient over time, and may comprise testing numerous samples collected over a number of months or years, in order to asses changes in the presence of aggregatory seeds, and in order to provide an improved diagnosis or assessment of risk. If the presence of aggregatory seeds in a sample from a subject increases over time, the risk of disease may increase, or the prognosis may become less positive.

In certain embodiments, the method of the invention is a method of monitoring disease progression in patients undergoing treatment for the disease. In such embodiments, the presence or levels of aggregatory seeds may be measured prior to administration of treatment and after administration of treatment, in order to assess the effectiveness of the treatment. If the presence or levels of aggregatory seeds decreases, treatment may continue. If the presence or levels of aggregatory seeds increases, treatment may be stopped or altered. The treatment may be directly targeted at preventing or reducing aggregation of the aggregatory protein, or the treatment may be targeted at a different mechanism involved in the disease process, such as an upstream mechanism, such as the production of the aggregatory protein. The methods of the invention will be useful for measuring the efficacy of treatments for diseases caused by the aggregation of a particular protein, including treatments that directly interact with the aggregatory protein and treatments that interact with other disease targets. When assessing treatments that interact with other targets or mechanisms involved in the disease process, the presence or levels of aggregatory seeds, as measured using the methods of the invention, may provide an indication of an individual's response to the treatment.

In the methods of the invention, the presence of aggregatory seeds in a clinical sample is correlated with the risk, presence, or severity of disease. In certain embodiments, the absolute or relative quantity of aggregatory seeds in the clinical sample is correlated with the risk, presence, or severity of disease. In certain embodiments, properties of aggregatory seeds are correlated with the risk, presence, or severity of disease. For example, certain species of seeds may be particularly aggregatory and seed more rapid aggregation of protein and therefore be associated with a very high risk for disease or with very severe disease. Also, certain species of seed may seed more disordered aggregates of protein and therefore be associated with a higher risk for disease or with more severe disease. As explained above, the properties of aggregatory seeds in a sample may be determined using the magnitude of a signal, such as total fluorescence signal, or the profile of a signal, such as a fluorescence lifetime signal or changes in emission and excitation spectra.

The level of the aggregatory seeds should be significantly different from that seen in a negative control. Advanced statistical tools (*e.g*. principal component analysis, unsupervised hierarchical clustering and linear modelling) can be used to determine whether two levels are the same or different.

If a method of the invention indicates that a subject has a conformational disease, further steps may then follow. For instance, the subject may undergo confirmatory diagnostic procedures, such as those involving physical inspection of the subject, and/or may be treated with therapeutic agent(s) suitable for treating the conformational disease.

The method of the invention is useful providing any subject with a diagnosis. In certain embodiments, the subject is at least 30 years old (e.g. >35, >40, >45, >50, >55, >60, >65, >70). In preferred embodiments, the subject is at least 50 years old.

The subject may be pre-symptomatic or may already be displaying clinical symptoms. For pre-symptomatic subjects the invention is useful for predicting that symptoms may develop in the future if no preventative action is taken. For subjects already displaying clinical symptoms, the invention may be used to confirm or resolve another diagnosis. The subject may already have begun treatment.

Because the invention can be implemented relative easily and cheaply, it is not restricted to being used in patients who are already suspected of having a conformational disease. Rather, it can be used to screen the general population or a high risk population e.g. subjects at least 30 years old, as listed above.

In alternative embodiments of the invention, the sample is an environmental sample, such as a sample collected from a field suspected of being contaminated with prion-containing animal faeces e.g. scrapie of sheep. Therefore, in certain embodiments, the method of the invention is used with a sample derived from or obtained from buildings or land used to house animals. In certain embodiments, the method of the invention is used with a sample suspected of comprising prion proteins, such as a food sample.

### Gel beads

The use of gel-forming agents and the formation of gel beads in the invention improve sensitivity of the assay and increase its functionality, because un-aggregated monomers and other factors can be washed away, whilst aggregates are retained, and because the gel beads can be readily transferred between assays that may be incompatible.

The gel bead 'cage' is formed by co-compartmentalising a gel-forming agent, such as liquid agarose, at elevated temperatures (such as 37 °C) and then later solidifying the interior of the droplet by dropping the temperature (such as to 25 °C) to initiate the sol-gel transition of the agarose matrix. Once solidified, these agarose microbeads retain large protein aggregates despite thorough washing and are amenable to a variety of standard laboratory manipulations, such as flow cytometry (using a flow cytometer or a fluorescence activated cell sorter, FACS). The confinement of seeds into agarose beads prevents diffusional loss of the fluorescence signal into the background medium and enables robust observation of the positive bead. In addition to the increase in sensitivity afforded by measuring in compartments rather than bulk phase, the washing procedure ensures that background fluorescence is removed in the fluorescent aggregate that can be detected with a better signal/noise ratio.

The gel-forming agent is an agent, for example a polymer such as polysaccharide or polypeptide, which can be solidified from a liquid into a gel, for example by alteration of conditions, such as heating, cooling, or altering pH. Alternatively, the gel-forming agent may be solidified into a gel through a cross-linking reaction. The cross-linking reaction may be initiated by delivering a crosslinker into the droplets or by treating the droplets with UV light. When the microdroplets have been transformed into gel beads (for example, by cooling), they are stable and porous.

Suitable gel forming agents include alginate, gelatine and agarose and other gels having a sol phase sufficiently fluid to move through the channels of a microfluidic device. Preferably agarose, which is a linear polymer made up of the repeating units of a disaccharide (D-galactose and 3, 6-anhydro-L-galactopyranose), is employed. In alternative embodiments, the gel forming agent is a polyacrylamide. The gel forming agent may be solidified into a bead by any convenient method, for example by changing the conditions. Preferably the hydrogel forming agent is solidified by altering the temperature, for example by cooling.

For example, when the agent is agarose, it may be solidified by reducing the temperature, for example below 25°C, below 20°C, below 15°C, below 10°C or below 5°C. The precise gelling temperature is dependent on the type of agarose and its concentration and may be easily determined by the skilled person. For example, 0.5% to 2% w/v of ultra-low melting point agarose Type IX-A(Sigma) has a gelling point of about 17°C. The agarose or other gel-forming agent may be solidified by cooling on ice.

Solidification of the agent within the microdroplet moulds the solidified gel into a bead. The population of solidified beads may be monodisperse. Alternatively, the population may be polydisperse.

Following emulsification and production of a gel bead, the aqueous microdroplets may be de-emulsified. In some embodiments, a deemulsification agent, for example a weak surfactant, may be added to the emulsion to separate the phases and the aqueous phase containing the beads removed. Any suitable mixture of oil and surfactant may be used. The deemulsification agent competes with the surfactant at the oil/water interface and causes it to collapse. Suitable weak surfactants include perfluorooctanol (PFO) and other fluorous compounds with a small hydrophilic group, if fluorinated oil is used, or a buffer containing SDS and Triton and other compounds with a carbon chain on one side and a small hydrophilic group on the other, if mineral oil is used. The deemulsification agent may be added to the emulsion and the mixture agitated, for example with a pipette. In other embodiments, the emulsion may be centrifuged to separate the phases and the aqueous phase containing the beads removed. Suitable techniques for the re-emulsification of gel beads are known in the art [52].

### Methods for assaying the effect of a test substance on aggregation

The use of microdroplets and gel beads in the methods of the invention allows sensitive analysis of the effect of a test substance on the aggregation of a protein. The method of the invention is particularly amenable to high-throughput screening of candidate therapeutics and identification of substances or compounds that are effective at inhibiting aggregation of a protein, because of the small reagent volumes used and the ease with which gel beads can be handled, for example using microfluidic and flow cytometry technology.

Candidate compounds or test substances may be small organic molecules, proteins, peptides, glycoproteins, nucleic acid molecules, antibodies (or fragments thereof) or any other suitable class of molecule.

The candidate compounds for use in the screening methods of the invention may be obtained using combinatorial library methods known in the art. For example, candidate compounds may be derived from biological libraries, peptoid libraries, spatially addressable parallel solid phase or solution phase libraries, or synthetic libraries. Examples of methods for the synthesis of molecular libraries are known to the skilled person [53-59]. Libraries of compounds can be presented in solution [60], or on beads [61], chips [62], bacteria or spores [63], plasmids [64] or on phage [65-68]. If the test substance is encoded by a nucleic acid, the method preferably comprises a step whereby the nucleic acid is expressed inside a microdroplet.

In certain embodiments of the method of the invention, a population of different test substances is compartmentalised into a plurality of microdroplets. In certain embodiments, following incubation of the microdroplets, transformation of the microdroplets into gel beads and detection of protein aggregates, certain beads are isolated. For example, using standard sorting equipment, beads comprising no or relatively few protein aggregates may be isolated. The test substances present in the isolated beads, which inhibit the formation of aggregates, may then be identified and optionally selected for further analysis or development.

In certain embodiments, the association with a test substance and its gel bead is maintained, to assist identification of effective test substances. The test substance may be retained in the gel bead by any convenient method. In certain embodiments, this is achieved by including particles in the microdroplets that comprise antibodies or tags or other moieties. For example, a library of test substances each comprising a first moiety could be assayed. This library could be compartmentalised into microdroplets with monomers of the aggregatory protein, a gel-forming liquid and particles comprising a second moiety with affinity for the first moiety. Following transformation of the microdroplets into gel beads, each gel bead will retain a protein aggregate formed under the influence of a particular test substance. Each gel bead will also retain the relevant particular test substance, which is associated with a particle via the first and second moieties. The particle used should be larger than the pore size of the gel bead. Such methods are particularly useful when small molecules are assayed.

The particle used to retain the test substance may be a nanoparticle or microparticle. The particle may be a magnetic bead. Suitable nano- or micro-particles are well-known in the art. For example, a particle may be coated with an agent, such as an antibody, which binds to the test substance. In some embodiments, the test substance comprises an epitope tag. A particle may be coated with an antibody or other binding member which binds to the epitope tag. Suitable epitope tags are well-known in the art including, for example, HA (Hemagglutinin), MRGS(H)6, DYKDDDDK (FLAGTM), T7-, S- (KETAAAKFERQHMDS), poly-Arg (R5-6), poly-His (H2-10), poly-Cys (C4) poly-Phe(F11) poly-Asp(D5-16), Strept-tag, Strept-tag II (WSHPQFEK), c-myc (EQKLISEEDL), Influenza-HA tag [69], Glu-Glu-Phe tag [70], Tag.100 (Qiagen; 12 aa tag derived from mammalian MAP kinase 2), Cruz tag 09™ (MKAEFRRQESDR, Santa Cruz Biotechnology Inc.) and Cruz tag 22™ (MRDALDRLDRLA, Santa Cruz Biotechnology Inc.). Known tag sequences are reviewed in reference 71. Strep-tag may be used in some preferred embodiments. Antibodies or other binding members may be covalently attached to the particle or may be indirectly attached, for example they may be biotinylated and may be attached to a streptavidin-coated particle through a biotin-streptavidin linkage using standard methods.

Suitable retention agents for retention of test substances include particles, for example non-synthetic particles such as viral particles, or synthetic particles, such as nano-or micro-particles, or polymers including cyclodextrins or dextrans, which are sufficiently large to be entrapped by the gel and unable to diffuse out of the bead.

Alternatively, the test substance may be retained through binding to the matrix of the gel bead itself, for example, through the use of a conjugated moiety with affinity for the matrix. The gel matrix may comprise a moiety (such as streptavidin or biotin, or alternative suitable binding members) that binds the test substance. Larger test substances may be retained in the gel bead as a result of their size.

Test substances, such as small molecule compounds, may be added in comparable ways, for example by using nanoparticles with chemically attached small molecule test substances that are encoded by an optical signal (e.g. quantum dot) or by a characteristic piece of DNA that can be PCR amplified and sequenced for identification [72].

In certain embodiments, an individual test substance is assayed in a population of microdroplets.

Once a candidate compound has been identified as a compound that is a modulator of protein aggregation, it may be desirable to perform further experiments to confirm the function of the compound *in vitro* or *in vivo*, for example using an animal model.

### BRIEF DESCRIPTION OF THE FIGURES

**Figure 1****:** Schematic demonstrating how microfluidic, but not bulk phase, techniques allow the detection of rare seeded aggregation events.

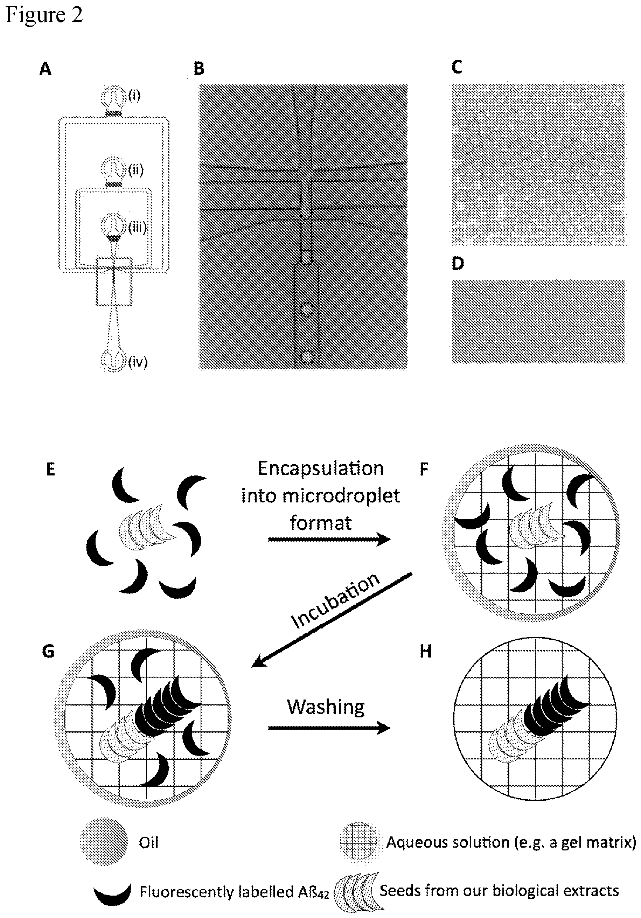

**Figure 2****:** Preparation of monodisperse gel beads. (A) shows the PDMS-chip in which monodisperse droplets are formed. The microfluidic device has three entries for carrier oil (i), agarose solution (ii) and sample spiked with, for example, Aβ peptide solution, such as fluorescent-labelled Aβ peptide solution (iii and E). The aqueous fluid phases (ii and iii) are mixed in the upper part of the junction shown in (B), and water-in-oil emulsion droplets (F) are made by 'break-off flow' when the aqueous stream meets the oil stream (i). Inlets (ii) and (iii) may be combined into one inlet. The emerging droplets (F) exit via outlet (iv). The agarose gel beads form when the water-in-oil emulsion droplets are left to cool from 37°C to a lower temperature (depending on the type of agarose, 4-25°C), triggering the gelation of agarose. When the gel beads have formed in the 'droplet template', the droplet boundary is broken-up to give agarose gel beads (D).
   The gel-containing aqueous droplets are then incubated to allow aggregation of the fluorescent reporter peptides onto the sample-derived seeds (G); this reaction occurs within the droplet and, when gel is present, within the substance of the gel matrix. Following the incubation step, the gel beads are released from the oil-droplet emulsion and suspended in aqueous washing buffer that permits the removal of unincorporated fluorescent-labelled peptide solution (H). The brightfield images in (C) and (D) show emulsion droplets (as they emerge from (iv)) and the washed beads, respectively.

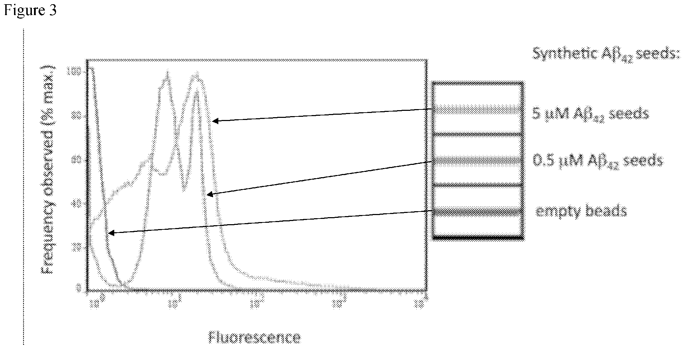

**Figure 3****:** Incubation of unlabelled seeds of synthetic Aβ₁₋₄₂ with fluorescently labelled monomeric Aβ₁₋₄₂ reporter peptides resulted in droplets with increased fluorescence intensity (5µM>0.5µM) as compared to beads without seeds (empty). Fluorescence analysis was performed in a flow cytometer (MoFlo FACS). The X axis represents the total fluorescence intensity per bead that passes through the cytometer. The Y axis is a normalised count of the number of beads with the particular level of fluorescence. The data normalisation means that for the three experimental conditions represented, differing numbers of total beads were analysed. However, the data were resized so that the various maximal peaks are all about the same height.

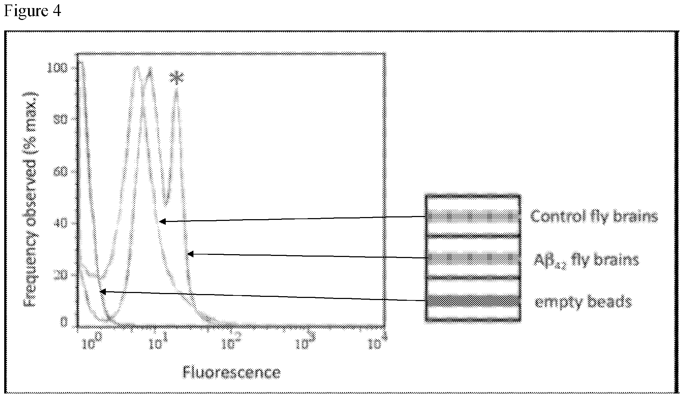

**Figure 4****:** Incubation of fluorescently labelled monomeric Aβ₁₋₄₂ reporter peptides with fly brain extracts result in a population of droplets with background fluorescence intensity (control fly brains line and A_{β1-42} fly brains line, 5-9 units). However, brain extracts from flies expressing Aβ₁₋₄₂ seed aggregation produce a novel, highly fluorescent subpopulation of beads (asterisk). A control reaction without brain extracts results in no fluorescence (0-1 unit, empty beads line). Fluorescence analysis was performed in a flow cytometer (MoFlo FACS).

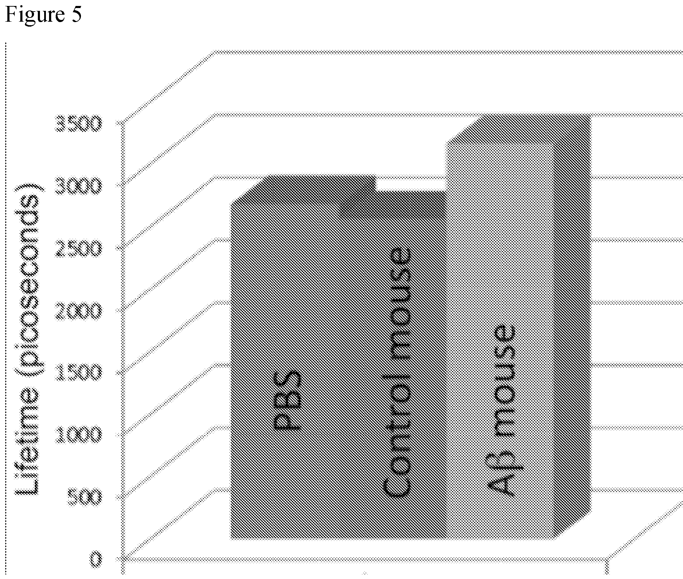

**Figure 5****:** The fluorescence lifetime of droplets containing control mouse brain extracts (central bar) is identical to droplets containing only PBS (left-hand bar). In contrast, Aβ-expressing mouse brain extracts result in the generation of droplets with a longer fluorescent lifetime, indicating less ordered structure (right-hand bar).

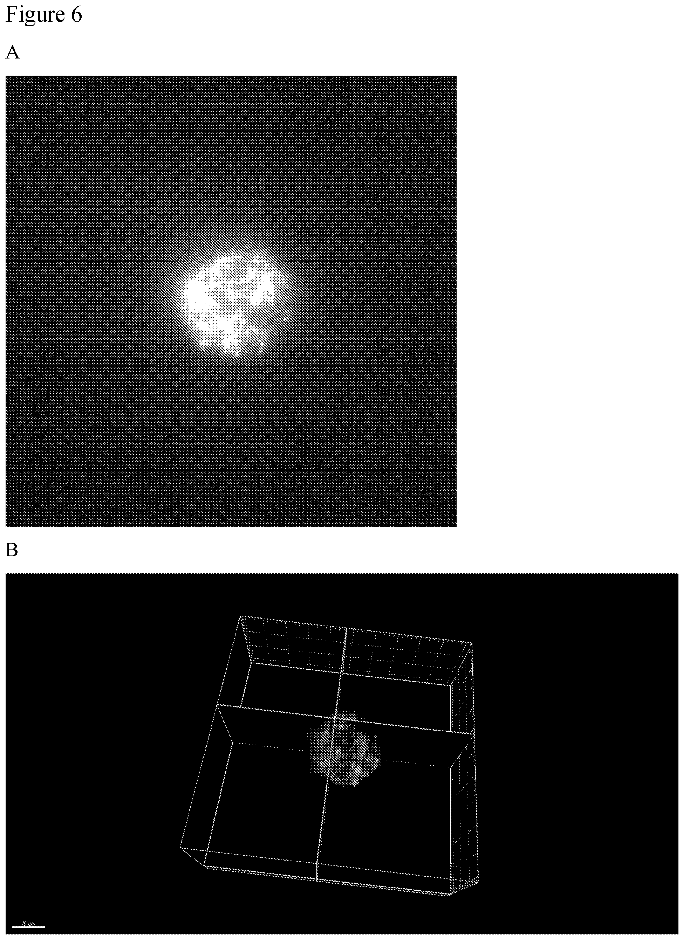

**Figure 6****:** Fluorescence image performed in half-TIRF mode showing Aβ₁₋₄₀ fibrils formed inside an agarose bead. (A) Fluorescence image performed in half-TIRF mode. (B) 3D volume view of the whole agarose bead.

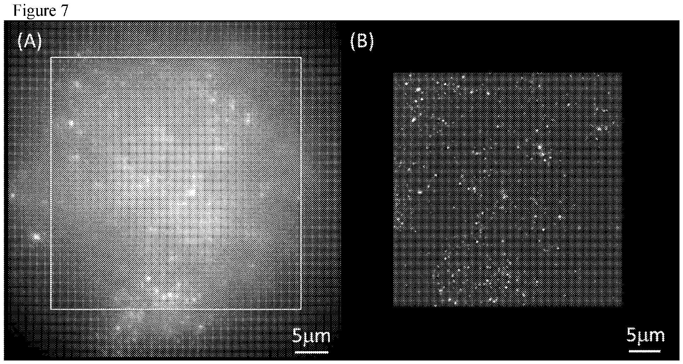

**Figure 7****:** (A) Standard widefield fluorescence image depicting a region within a 45 µm diameter agarose bead containing Aβ₁₋₄₀-Fililyte488. The aggregation was allowed to proceed for 24 hrs before the image was taken. (B) Deconvolved image of the same region as shown in (A) imaged and deconvolved using structured illumination microscopy. The image data was obtained from a stack of 16 image slices spaced at 500 nm.

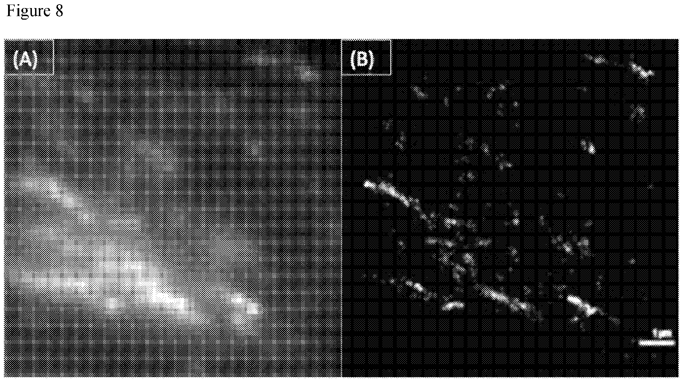

**Figure 8****:** dSTORM imaging of a selected area inside an agarose bead with Aβ₁₋₄₀-Hilyte647 incubated for 24hrs. (A) Fluorescence image of Aβ₁₋₄₀ fibrils formed inside an agarose beads. (B) dSTORM image performed in half-TIRF mode corresponding to the fluorescence image in (A).

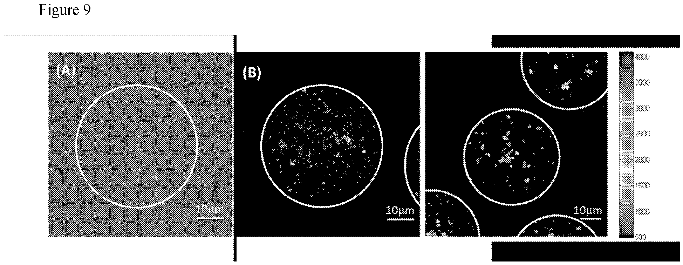

**Figure 9****:** Fluorescence lifetime images of agarose beads prepared with different forms of Aβ₁₋₄₀ inside. (A) Agarose beads mixed with Aβ₁₋₄₀ peptide monomers and immediately washed after bead formation, (B) Agarose beads were mixed with HF488Aβ 1-40 peptide that had been incubated for 24 hours prior to droplet formation. After droplet and bead formation the oil/water interface was broken while monomers and small size aggregates of HF488 Aβ 1-40 were washed out of the agarose beads. (C) Aβ₁₋₄₀ peptide incubated in agarose beads for 24 hours and then washed. Conditions: 37°C, Napi pH7.4.

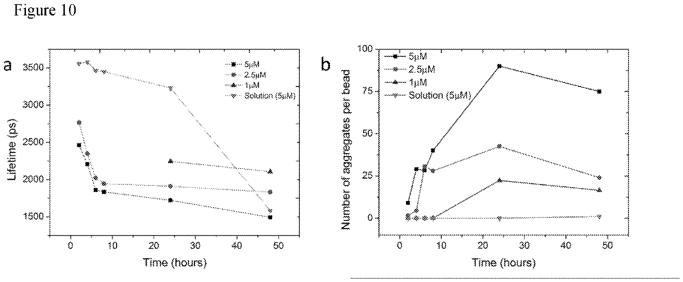

**Figure 10****:** Confocal fluorescence lifetime images of beads containing HiLyte 488-labelled Aβ₁₋₄₀ at various concentrations and conditions. (A) The decrease in fluorescence lifetime, averaged over each bead, is shown for 3 different starting concentrations (1 µM, 2.5 µM and 5 µM Aβ₁₋₄₀; upward pointing triangles, circles and squares, respectively) and at different aggregation time points. Measurements are also shown for a concentration of 5 µM Aβ₁₋₄₀ in bulk solution (downward pointing triangles). Aggregation occurs more quickly in the beads compared to in solution and the speed of aggregation is concentration dependent. (B) The relative 'aggregate count' per bead is shown, defined as the number of pixels containing low lifetimes compared to those containing high lifetimes Conditions: 37°C, Napi pH 7.4.

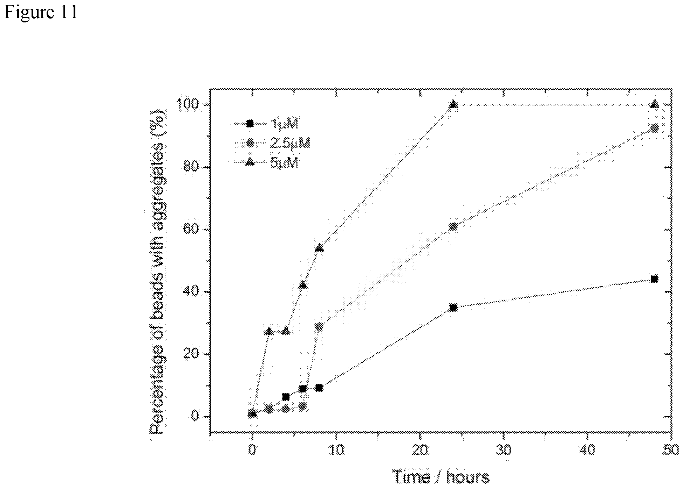

**Figure 11****:** Flow cytometry analysis of Aβ₁₋₄₀ peptide beads permits the time course of aggregation to be quantified with high sensitivity. The graph shows that the percentage of beads containing fluorescent aggregates of Aβ₁₋₄₀ increases over time for three different peptide concentrations (squares: 1 µM, circles: 2.5 µM, triangles: 5 µM).

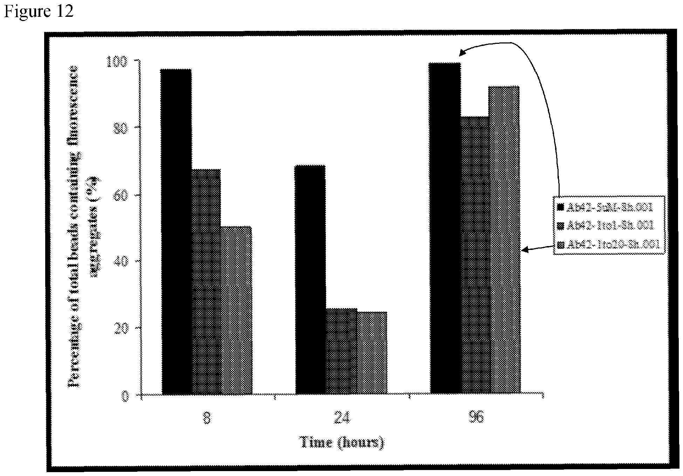

**Figure 12****:** Inhibition of aggregation by *scyllo*-inositol. *Scyllo*-inositol and amyloid monomers (Aβ₁₋₄₂) were compartmentalised in droplets and incubated at ratios of amyloid:inhibitor of 1:1 (central bar at each time point) or 1:20 (right-hand bar at each time point), respectively. The left-hand bar at each time point represents a control with no *scyllo*-inositol. The bar diagram displays the results of the flow cytometric analysis of HF488Aβ₁₋₄₂ in the presence or absence of *scyllo*-inositol.

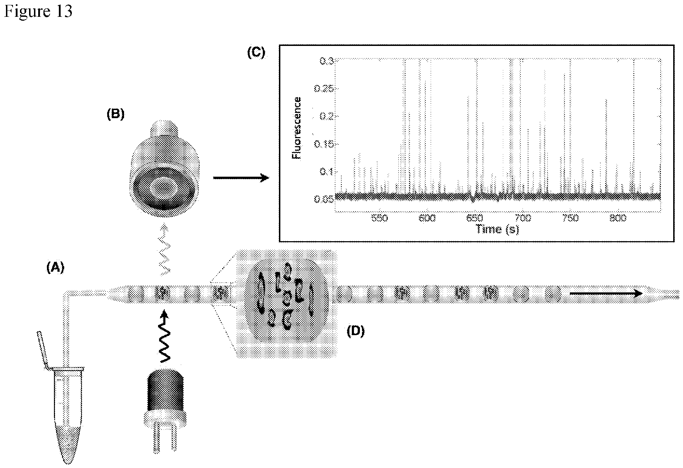

**Figure 13****:** *Scheme of a fluorescence detection system for seeded formation of aggregates in beads.* The amyloid-gel-beads (A) are sipped through a capillary that passes through the laser beam of a planar laser-induced fluorescence (PLIF) device represented by (B). A fluorescent peak for each droplet is quantified using a fiber-optics system (C). The graph represents data from the screening of Jurkat cells that exhibit fluorescent aggregates around the nucleus (lower peaks) mixed with fluorescent spheres (taller peaks). The fluorescent aggregates in Jurkat cells are similar in size to amyloid aggregates detected in gel-beads, schematically represented (D).

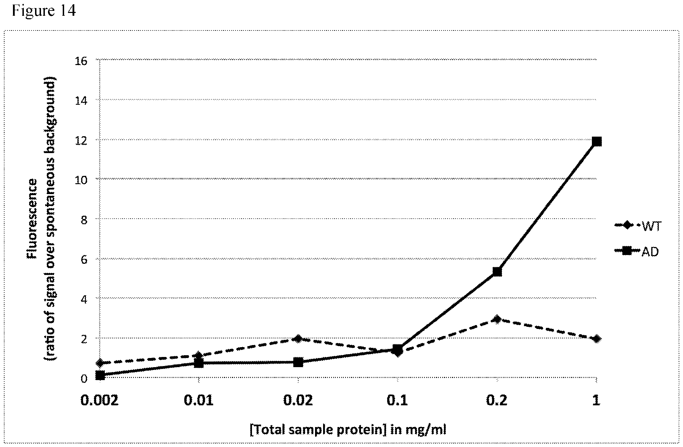

**Figure 14****:** *Comparison of the assay reading for diseased (AD) and non-diseased (WT) murine brain samples over background.* The figure shows the differences between AD and WT samples from murine brain at various concentrations. At concentrations of total protein over 0.2 mg/ml, there is a significant difference in signal.

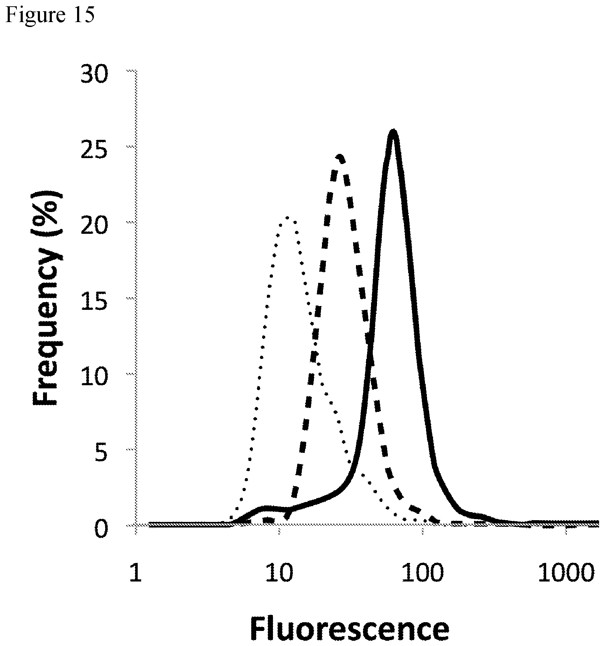

**Figure 15****:** *Detection of seeded aggregation of Aβ42*, *as a marker of AD, in murine serum.* Alzheimer murine models (aged CRND8 mice, unbroken line) serum was compared to age-matched, non-diseased littermate control (dashed line) serum. The frequency of high fluorescence beads is greater for the AD serum than for the WT serum. The background trace (dotted lines, serum omitted) represents the spontaneous aggregation signal.

### EXAMPLES

### Example 1 - Preparation of gel beads

### Device fabrication

The microfluidic devices used in this study were constructed using conventional soft lithographic techniques (Fig. 2). Briefly, a master was prepared from SU-8 2025 (Microchem Corp.) on a silicon wafer (Compart Technology Ltd.). Slygard 184 (Dow Corning) was mixed in a 10:1 (w/w) ratio of resin to crosslinker and then poured over the master. A thickness of 50 µm was achieved by adjusting the spin coater velocities in two spinning phases: initially with 500 rpm for 5 s at an acceleration of 300 rpm/s and subsequently with 1650 rpm for 40 s. at 300 rpm/s. After degassing, the device was cured for six hours at 65 °C, and PDMS was cut and peeled off the master. The device (total length approximately 1-5 cm) was exposed to air plasma for 30 seconds (Femto Diener) and sealed onto a standard microscope glass slide (Agar Scientific). The channels were then rendered hydrophobic by baking at 65 °C for 10-12 hours before use. Electrodes were fabricated afterwards by placing the device on a hotplate and heating to 130°C.

### Formation of gel beads

A microfluidic droplet generator with a flow focusing geometry [73] was used to generate water-in-oil droplets. The contents of the droplets were determined by the content of the supply stream: peptide buffer solution was mixed with agarose gel solution and trapped by HFE7500 oil containing a surfactant, e.g. 0.5% (w/w) fluorous tri-block copolymers [74,75]. Alternatively, a number of alternative oil/surfactant combinations can be used, e.g. mineral oil containing about 3% Abil EM 90,or mineral oil (95% w/w) containing about 4.5% (w/w) Span 80 and about 0.5% (w/w) Tween 80. The emulsion droplets were produced at around a 4.4 kHz frequency. Afterwards, the emulsion droplets were collected and 'immortalised' by cooling (e.g. on ice or in a water bath): here an emulsion droplet was created containing liquid agarose and cooled to trigger the transformation of agarose-containing droplets into gel beads. Subsequently, the emulsion was broken by removing the surfactant layer coating of the beads by adding 1H,1H,2H,2H-perfluorooctanol (97%) oil. In the case of mineral oil/non-fluorous surfactant combinations the emulsion can, for example, be broken by freezing the emulsion in a microtube and centrifuging at maximum speed for 5 minutes. The aqueous phase will be below the oil layer.

Eventually, the bare gel beads were washed three times with Napi (Sodium phosphate) buffer (pH 7.4, 50 µM sodium phosphate) and stored in the buffer until an optical assay was carried out. In this final step the non-aggregated peptides were extracted from the beads. In contrast, the large aggregates are trapped inside the beads as they are too bulky to leave the agarose gel matrix. The agarose beads can retain aggregates, but also allow for adding and extraction of components through its gel matrix, which provides a platform for studying aggregates at a variety of settings. A schematic illustrating the formation of the beads is provided in Figure 2.

Therefore, monodisperse droplets are produced in a microfluidic device and loaded with all reagents. After offline incubation for a defined aggregation time interval, the droplets can be retrieved and incubated on ice. The temperature drop prevents further aggregation reactions from taking place and effectively inhibits aggregation. Furthermore, the lowered incubation temperature is below the gelling point of agarose and triggers the transformation of the droplets into porous and stable beads, which is completed within 2 minutes or less. The bead structure persists even upon subsequent temperature elevation to ambient conditions and after removing the droplet oil boundary through addition of 1H,1H,2H,2H-perfluorooctanol (97%) oil or, the case of mineral oil/no-fluorous surfactant combinations by phase separation by centrifugation. The porous structure of the agarose bead matrix serves to retain aggregated amyloid, whilst small molecule components and buffer are free to enter or exit the bead. The beads thus become a 'time capsule' for aggregated product, which can be subjected to a number of downstream analysis methods. The ability to wash resulting agarose beads and remove small oligomers and molecules enhances the sensitivity of fluorescence-based assays because the relative signal from trapped amyloids increases while background from unaggregated material is removed. The small droplet volumes (∼ 50 pL) provide for economical experiments and multiple redundancy: For example, a total reaction volume of 1 µL can generate up to 10⁵ identical droplets and beads, which constitutes an enormous pool for either averaging techniques or individual analysis.

### Example 2 - Detection of aggregatory seeds of Aβ in microfluidic droplets using total fluorescence

This example demonstrates the successful detection of synthetic aggregatory seeds of Aβ using microfluidic droplet gel beads. Synthetic seeds were generated from unlabelled Aβ₁₋₄₂ that was incubated for 10 days (50 µM, Napi buffer, pH 7.4). These seeds were then incubated with fluorescently labelled Aβ₁₋₄₂ monomers in microfluidic droplets. The total fluorescence of the droplets was measured using FACS analysis and was compared to the fluorescence of droplets incubated without synthetic seeds (i.e. droplets that comprised fluorescently labelled monomer only). As shown in Figure 3, droplets containing synthetic seeds (µM and 0.5µM) exhibited increased fluorescence intensity compared to beads that did not comprise seeds (empty beads). Therefore, the presence of seeds results in a population of droplets with high fluorescence intensity, representing those droplets in which rapid production of amyloid fibres results from the presence of a seed. Populations of droplets comprising seeds can be distinguished from populations that do not comprise seeds.

### Example 3 - Detection of aggregatory seeds of Aβ in fly brain samples using total fluorescence

This example demonstrates the successful detection of aggregatory seeds of Aβ in samples obtained from brain extracts of a *Drosophila* model of Alzheimer's disease [76,77]. As in Example 2, the total population of beads was considered and the distribution of fluorescence intensities was measured.

This experiment used transgenic flies expressing the Aβ₁₋₄₂ peptide. Wild type flies were used as a control. Brain extracts were prepared using the protocol outlined below.
1. Male flies were collected in a 1.5 ml snap-top tube at 5, 10 and 25 days old. Flies were snap-frozen for 30 sec in liquid nitrogen and then vortexed for 30 sec.
2. Step 1 was repeated a further 2 times.
3. The contents of the snap-top tube were placed on a weighing boat on a bed of dry ice and the heads were separated from the rest of the bodies.
4. The heads were collected in fresh 1.5 ml snap-top tubes (30-50 heads for control and 30-50 heads for Aβ flies).
5. Fly heads were homogenized using a disposable plastic pestle in 15 µl of PBS + 0.05% (w/v) SDS.
6. The samples were then sonicated in an ice cold water bath at maximum intensity for 8 min.
7. The samples were then centrifuged at 3000g for 1 min.
8. The protein concentration of the extracts was determined using the BioRad protein assay (or nanodrop) and adjusted to 3 mg/ml.
9. The supernatant was collected and stored at -80°C.

The fly brain samples were incubated with fluorescently labelled Aβ₁₋₄₂ monomers in microfluidic droplets. The total fluorescence of the droplets was measured using FACS analysis. As shown in Example 2, seeded aggregation of the fluorescently labelled peptide should be accelerated when compared to non-seeded aggregation, so it was expected that beads containing an aggregatory seed (from the non-labelled sample derived from Aβ₁₋₄₂ flies) should accumulate more aggregate within a particular time and so have a higher fluorescence signal. Figure 4 demonstrates that in the absence of any fly brain extract there is very little fluorescence signal retained within most beads (empty beads). In contrast, the addition of control fly (wild type) brain extracts results in a significant right shift in the population (control fly brains line), which suggests non-specific binding to the agarose and the fluorescent Aβ₁₋₄₂ peptide, or else some non-specific seeding activity. However, when brain extracts from flies expressing Aβ₁₋₄₂ are added to assay we see a novel, highly fluorescent subpopulation (Aβ₄₂ fly brains line, asterisk). This represents beads that contain one (or potentially more) seeds and so accumulate more fluorescent aggregate with time as compared to unseeded beads incubated with wild type brain extract.

We have observed that the signal to- noise ratio for the assay is higher as the extracts are diluted and this is a powerful characteristic of the digital signal that can be generated by microfluidic techniques.

### Example 4 - Detection of aggregatory seeds of Aβ in fly brain and mouse brain samples using fluorescence lifetime microscopy

A second way that the production of seeded aggregates can be measured is using fluorescence lifetime imaging. This technique relies on measurement of how the fluorescence signal declines with time after a very brief excitation. Normally a monomeric peptide in solution with the HiLyte fluorescent dye would have a lifetime of approximately 4000 picoseconds. When the peptide aggregates the kinetics of fluorescence decay are accelerated such that the half-life may decrease to about 2500 ps.

Between these extremes we can assess the structural content of the seeded aggregates. Interestingly, we find that spontaneous aggregation (spiked with PBS only) results in a low half-life that is indistinguishable from aggregates resulting from incubation with control mouse brain, while aggregates seeded from diseased mouse brain have a significantly longer half-life. This means that the diseased-seeded aggregates are less structured than the spontaneous aggregates, and this demonstrates a further way in which samples comprising aggregatory seeds can be distinguished from samples that do not comprises aggregatory seeds.

Brain extracts from two strains of mice were made in PBS using the protocol below.
1. The Hippocampal and cortex mouse brain tissues (from littermate control and APP-expressing CRND8 mice [78]) were taken out from -80°C freezers and kept on ice.
2. The tissues were weighed using disposable weighing boats and cut it into small pieces using the sterile scalpels.
3. The tissues were homogenized using a Douce homogeniser in the fume cupboard wearing lab coat, gloves and mask to avoid the contact with the skin and inhalation of particles.
4. The control sample was homogenized first. The tissue was put into homogeniser and suspend in autoclaved PBS (10% PBS w/v, e.g. 10 ml PBS for 1 g tissue).
5. The material was collected into an Eppendorf tube and vortexed for 2 min.
6. The homogenizer was washed and the disease sample (APP-expressing) was prepared repeating the steps 4 and 5.
7. After processing the samples, the homogenizer was washed with 2ml 1X PBS and used as control sample.
8. The samples were sonicated in an ice cold water bath with the tubes sealed at maximum intensity for 8 min.
9. The samples were centrifuged at 3,000 g for 5 min.
10. The middle layer was collected in a fresh Eppendorf tube using pipette.
11. The samples were again centrifuged at 3000g to remove any debris left.
12. The supernatant was collected in fresh tube and stored at -80°C (10 % w/v extract).

The first strain was a normal laboratory mouse and the second littermate control mouse was derived from the same strain but was transgenic for a disease-linked variant of human amyloid precursor protein (APP). As a consequence the transgenic mice accumulate Aβ aggregates, show AD-related phenotypes and are used as a model of AD. We compared the biophysical characteristics of the aggregates that were generated when brain extracts were mixed with fluorescence-labelled Aβ₁₋₄₂ peptides within the agarose-containing microdroplets. Fluorescence lifetime microscopy was used to inspect the structure of the aggregates that were retained within the microbeads following the washing step. As shown in Figure 5, it was found that the fluorescence lifetime of aggregates that are formed in the presence of control mouse brain extracts was identical to the lifetime of aggregates formed in the presence of the vehicle, PBS. In contrast, there was a marked prolongation in the fluorescence lifetime of aggregates in beads exposed to the diseased-mouse extracts, suggesting that in this case the Aβ species generated were less ordered.

### Example 5 - Imaging of aggregates in agarose beads

To follow the spatial distribution of amyloid nucleation and growth within the bead structure we used highly inclined illumination [79], and epi-fluorescence microscopy, as well as direct stochastic Optical Reconstruction Microscopy (dSTORM) [42,43] to look at distribution of the aggregates. The latter is an optical super resolution imaging technique that permits features to be resolved at a resolution down to 20 nm [80]. Figure 6A shows a single image slice obtained of HiLyte488-Aβ₁₋₄₀ aggregates that were incubated at 5 µM for 24 hours in the agarose beads. The image was obtained with highly inclined illumination microscopy, HiLo [42,79] and represents a slice through the centre of the bead. Figure 6B shows a 3D rendering of the protein distribution within the beads. It is clear that protein accumulates in channel like features throughout the bead structure, reflecting the porous structure of the agarose gel matrix. We performed TEM imaging and used the method of Griess [81,82] to estimate pore size in the present agarose beads to be in the 200 nm range. This is similar to what has been reported previously for similar systems [83]. Because monomers are removed after the washing step it appears from Figure 6 that aggregates are retained in the channel structure; indeed, it can be hypothesised that the channel structure has a significant influence on the kinetics of aggregation, a topic that is explored in a subsequent section.

Figure 7 shows structured illumination microscopy (SIM), a superresolution technique that permits features to be imaged at a resolution smaller than the wavelength of light [84,85]. We used an OMX microscope (Applied Precision, USA) to visualise the locations of aggregates inside the bead through sequential recording of optical image slices through the beads (fluorescence images in 16 µm slices from a 45 µm diameter bead were obtained by OMX) with a structured illumination pattern and subsequent image reconstruction. The results are shown in Figure 7. Figure 7(A) shows a standard widefield image of a slice within the bead. In Figure 7(B) the same region is visualised using SIM. Clearly both resolution and contrast are greatly enhanced by the technique. Aggregates of Aβ₁₋₄₀-Hilyte488 are distinguishable within the agarose matrix and solution. The average pore diameter in gels formed from 0.6% w/w agarose has previously been reported to range from 100 to 300 nm [83]. Thus, the agarose 'cage' structure is effective at retaining aggregates larger than this size. Monomers are removed after the washing step, so the aggregates are effectively 'frozen' in their macromolecular state upon agarose solidification. The random distribution of aggregates inside the bead suggests that aggregation takes place within the voids of the beads. No preferential aggregation was seen to take place at the oil/water interface. The confinement of aggregates and monomers in the pore may affect rates of nucleation and growth, similar to what is observed in crowded environments, for example in cells [37]. The growth kinetics in beads is studied in detail in subsequent sections.

As the resolution of widefield HiLo is not sufficient to resolve individual aggregates, we performed high resolution dSTORM experiments on Alexa647-labelled Aβ₁₋₄₀ under similar conditions. It has been reported that conventional fluorophores can be used as efficient photoswitchable fluorescent probes [86]. By irradiation with light of different wavelengths without an activator fluorophore, there can be a reversible cycle for these probes between a fluorescent and a dark state. This is the principle of stochastic optical reconstruction microscopy (STORM) with nanometer accuracy and subdiffraction-resolution images. Significant improvement of the Aβ₁₋₄₂ structure observation has been proven by dSTORM imaging [87]. For detecting the detailed structure of the aggregates formed inside agarose beads, super-resolution fluorescence imaging was performed by total internal reflection fluorescence (TIRF) configuration equipped with an oil-immersion objective [88]. In order to visualise the clear structure of aggregates inside the agarose bead, a smaller area was selected and magnified (Figure 8).

For the preparation of the dSTORM images the photo switching buffer was prepared and injected into beads reservoir. Oxygen contamination to the switching buffer was prevented by sealing the reservoirs before the imaging process was initiated. 5000 images of a certain area of an agarose bead were recorded. Based on a custom made Matlab code, the images were reconstructed. Clear structure and different length of aggregates can be observed down to a size of ∼100nm.

### Example 6 - Lifetime fluorescence analysis of aggregation

This example demonstrates the use of fluorescence lifetime imaging to assess and monitor the aggregation state of amyloid in a bead, which is useful for screening.

Lifetime shifting of Hilyte488-labelled Aβ₁₋₄₀ was monitored on a TCSPC (Time-Correlated Single Photon Counting) confocal microscope setup. Agarose beads were imaged by a 60 X 0.25 oil objective (Leica). The laser beam was generated by a super continuum source (20 mHz) and excitation was set to 488 nm in order to excite the tagged fluorophore Hilyte488. The emission was filtered by a 515 nm longpass filter. In order to probe the lowest possible concentration of Aβ₁₋₄₀ that could still lead to detectable aggregation, three different concentrations of Aβ₁₋₄₀ were prepared in Napi buffer solution (5 µM, 2.5 µM and 1 µM) and were mixed with 1.5 % agarose solution. The 0.75 % agarose emulsion samples were incubated at 37°C for 2, 4, 6, 8, 24 and 48 hours, respectively. Afterwards, the emulsion droplets were immobilised, broken and washed, as described above, and used for TCSPC analysis.

The fluorescence lifetime of bead-caged amyloid samples was analysed by TCSPC using a custom designed microscopy set-up [89]. We have previously shown that the fluorescence lifetime of fluorescently tagged amyloid aggregates is a sensitive readout of aggregation state and decreasing lifetimes are indicative of increasing degrees of aggregation [37]. A major motivation for the current work was to establish lifetime imaging in conjunction with microdroplet technology as a new platform to investigate molecular mechanisms of aggregation and for the screening of potential aggregation inhibiting small molecule agents. Figure 10 shows confocal fluorescence lifetime images of beads containing HiLyte 488-labelled Aβ₁₋₄₀ at various concentrations and conditions. Fig 9A shows agarose beads mixed with monomeric solutions of Aβ₁₋₄₀. Clearly the monomer infuses the agarose beads and fluorescence signal is observed both from the inside and outside of the bead, whose contour is shown. The homogenous blue colour indicates a mean lifetime of around 4000 ps, close to the value of the isolated fluorophore label. Washing the bead with Napi pH7.4 buffer completely removes all peptide from the beads such that no fluorescence can be observed (data not shown). Fig 9B shows aggregates of 5µM HiLyte 488 labelled Aβ₁₋₄₀ appearing in bulk solution after 24 hrs incubation. Agarose beads were added to the aggregate containing solution and the mixture subjected to a washing step to remove monomers and small oligomers of peptide. It is clear that the bead retains aggregates whose lifetime is significantly lower than that of the monomer mixture (Fig 9(A)). Signal outside of the bead is non-existent, and almost no background from monomers is observed from within the beads (Fig 9(B)). This result demonstrates the aggregate-retaining capacity of the droplet cages. Figure 9(C) shows aggregates obtained under similar conditions as in Fig 9(B) but this time, the aggregation was allowed to proceed in the presence of agarose beads. Again, the mixture was washed after 24 hours in buffer solution and again it is apparent that the cages act as efficient retainers for the aggregated species, rejecting monomeric or small oligomeric forms of the peptide. In contrast to the aggregates produced in bulk solution, lifetimes dropped from around 3000 ps to around 2500 ps, which indicates that aggregation proceeds more efficiently in beads compared to bulk solution.

To investigate this more quantitatively, the aggregation time course was measured for different concentrations of peptides in beads and compared to bulk measurements (Fig 10). Samples of increasingly mature Aβ₁₋₄₀ amyloid exhibited lifetimes that decreased from 3500 to 1500 ps (Fig. 10(A)). This range of lifetime decrease is identical to what had previously been observed in bulk solution via fluorescence-lifetime imaging microscopy (FLIM) [37] and indicates that the proteins attain similar structural forms in beads and in bulk [87]. However, in droplets the observed decrease occurs much more rapidly over time, supporting our hypothesis that beads accelerate protein self-assembly reactions. In Figure 10(A) the decrease in fluorescence lifetime, averaged over each bead, is shown for 3 different starting concentrations (1-5 µM Aβ₁₋₄₀, upward pointing triangles, circles and squares, respectively) and at different aggregation time points. Measurements are also shown for a concentration of 5 µM Aβ₁₋₄₀ in bulk solution (downward pointing triangles). It is clear that, the aggregation time course occurs much more quickly in the beads compared to in solution and the speed of aggregation is furthermore concentration dependent. In Figure 10(B), the relative 'aggregate count' per bead is shown, defined as the number of pixels containing low lifetimes compared to those containing high lifetimes (i.e. above or below a threshold of 3000 ps). For similar starting concentrations the aggregate count is significantly higher in the beads compared to what is observed in bulk. These trends are partially caused by the fact that the bead cages act as traps for larger aggregate sizes whilst monomers are washed away. This improves signal to noise ratio compared to measurements in solution which are dominated by the (long) lifetime of soluble (mainly monomeric) Aβ₁₋₄₀. The assay reliably detects aggregates of Aβ₁₋₄₀ in concentrations of peptide as low as 1 µM, far below what is possible in bulk. The number of aggregates was decreasing from 24 hours to 48 hours, indicative of aggregate 'coalescence' or possibly fibril formation. Especially aggregates with intermediate lifetimes that are hard to detect in solution can be accessed more quickly. These attributes are favourable for potential use in screening applications permitting sensitive and rapid testing of e.g. potential aggregation inhibiting agents that are the focus of intense drug discovery efforts.

### Example 7 - Use of flow cytometry to detect and follow spontaneous aggregation of Aβ₁₋₄₀

This example describes the use of gel beads to measure spontaneous (non-seeded) aggregation. The number of protein aggregates in beads arising from the HiLyte488 labelled Aβ₁₋₄₀ peptide was analysed by flow cytometry, and quantified via measurement of ensuing fluorescence from the aggregates retained within bead cages. At a droplet generation rate of 4.4 KHz a population of approximately 5.3 × 10⁶ droplets was obtained over 20 min, from which aliquots of approximately 2 × 10⁵ droplets were withdrawn. The droplets were contained throughout the aggregation time course (up to 96 hours) and analysed at periodic intervals. Figure 11 shows the increase in aggregate number appearing over time. It is notable that Aβ₁₋₄₀ peptide can be seen to aggregate at concentrations as low as 1 µM, where significant signal is obtained at >24hrs (see Fig. 11, black squares). This concentration is far too low for Aβ₁₋₄₀ to form aggregates in bulk solution under similar conditions. It is possible that the porous bead structure accelerates the aggregation process through geometric confinement which is more akin to the crowded environments presented in cells under physiological conditions (where aggregation is frequently observed at low concentrations of peptide). Further analysis (see subsequent sections) revealed that Aβ₁₋₄₀ is indeed present in aggregate form in the beads. These findings attest to the great sensitivity of the bead based aggregation assay which is superior to that of the most sensitive reported methods [89-91], which do not permit observations below peptide concentrations around 10 µM. In traditional bulk systems, reliable readouts have not been reported for Aβ₁₋₄₀ peptide concentrations below 50 µM.

### FACS analysis

Since the processed gel beads were stored in aqueous buffer solution, they could be tested via flow cytometry or FACS, a technique requiring aqueous carrier fluid, in order to assess the aggregation process and compare different intensity of aggregates inside agarose beads. Incubated and washed agarose beads with Hilyte488-labelled Aβ₁₋₄₀ aggregates were sent to FACS and the beads with fluorescent aggregates were counted. Empty beads with only agarose and no Aβ₁₋₄₀ were also prepared as a control group for FACS testing. The percentage of beads containing aggregates was calculated using the program Summit. Conditions: 37°C, Napi pH7.4.

### Example 8 - Screening inhibitors of aggregation

This example demonstrates the use of the assays of the invention to successfully identify a known inhibitor of Aβ aggregation. Aggregatory Aβ₁₋₄₂ monomers were incubated in gel beads with *scyllo*-inositol, which is a known inhibitor of Aβ aggregation. As shown in Figure 12, the inhibition of aggregation by *scyllo*-inositol was detected.

Therefore, the assays of the invention are capable of identifying a known inhibitor, and will be useful for identifying further inhibitors of aggregation.

### Example 9 - Fibre optic detection of fluorescence

The assays and methods of the invention encompass simplified systems wherein droplet generation and fluorescence detection are integrated. For example, the chip device for droplet and bead formation may be built in harder plastic or glass (instead of PDMS). In addition, a fibre optics-based system can be used to detect fluorescent aggregates in beads as they pass through a capillary to be detected with a portable laser-induced fluorescence (PLIF) reader. Figure 13 provides an outline of such a system. It is possible to contain thousands of beads in a capillary and move them past the detector for measurement when desired. This is convenient for taking time courses, and identifying when sufficient numbers of aggregates have arisen, so that large scale analysis can be performed.

### Example 10 - Probing disease progression

The assays and methods of the invention can be used for monitoring disease progression. The *Drosophila* model of Alzheimer's disease used in the previous examples provides an important link between link between experimentally observed and computationally predicted aggregation behaviour and neurotoxicity [92], based on the ability to set up a disease cycle from presymptomatic through moderate disease and to death within 30 days. Several neurological assays are available (e.g. locomotor performance, reduced longevity and histological indicators [93,94]) and measurements of aggregatory seeds obtained with the droplet bead assay can be correlated with these known indicators.

Transgenic Drosophila expressing various isoforms of the Aβ peptide (1-40, 1-42 and the arctic variant of 1-42) are generated and aged up to 40 days. At the required age aliquots of 50 flies are anaesthetized briefly in CO₂ and then frozen in liquid nitrogen in a snap top tube. Following decapitation by vortexing while frozen, the heads are collected and homogenized in 50 µl ice-cold PBS using a disposable pestle. The homogenate is centrifuged at 10,000 g for 5 min and the pellet is discarded. The protein concentration of the supernatant is estimated using a nanodrop (OD280 nm) and PBS added to make standard extracts of 3 mg/ml. The control samples include: similar flies not expressing a transgene or flies expressing another unrelated neurotoxic aggregation-prone polypeptide such as polyQ [95]. In other controls the Aβ- expressing fly extracts are immunodepleted using two monoclonal antibodies (4G8 and 6E10) to control for non-Aβ effects. Non-transgenic fly extracts are also mock-immunodepleted. The measurements using the microfluidic assays of the invention are correlated with disease phenotypes in the fly, including biochemical markers, such as brain Aβ levels and markers of oxidative stress, and phenotype measurements, such as locomotor assays and mortality, in particular the gradient of the mortality trajectory plot (log of the chance of dying vs. time).

Measurements of aggregatory seeds obtained with the droplet bead assay can also be correlated with disease progression in a murine model of neurodegenerative disease. While for flies brain extracts are the only accessible sample, a mouse model gives access to a variety of different samples that can be analysed in droplet bead assays. For example, a CRND8 mouse model of Alzheimer's disease is available. Multiple tissue extracts (brain (see, for example, Figure 14), serum (see, for example, Figure 15) and urine) are tested and the assays of the invention are used to detect the presence of aggregatory seeds in these samples. Murine brain and serum samples are treated analogously to the fly brain samples described above, resulting in stock solutions of 3 mg/ml protein. The urine samples are centrifuged and the supernatant diluted 9:1 with 10x PBS. 10 sets of CRND8 mouse tissues and a similar number from their non-transgenic littermates are used. The control samples for the murine experiments are similar to the fly controls. In particular, the CRND8 extracts are immunodepleted, producing a loss of signal. In both the fly and mouse experiments an Aβ-dependent population of high-fluorescence intensity droplets that have a relatively high fluorescence lifetime is measured. These results are correlated with phenotypic staging in the organisms. In particular, it is determined how early is the signal apparent and how does it develop as the phenotypes become more severe.

The measurements of aggregatory seeds obtained with the droplet bead assay can also be correlated with progression of human disease. In the same way as for the flies and mice, extracts from frozen post-mortem brain tissue (hippocampus) from 10 AD patients (medium-severe) and 10 age- and sex-matched non-demented controls are collected. CSF samples from the same patients are prepared by the same method used for the mouse urine samples. The assays of the invention are used to detect aggregatory seeds in the brains of subjects with Alzheimer's disease, and the amount of aggregatory seeds is correlated with disease progression, as in the animal models above.

### Example 11 - Detection of aggregatory seeds of Aβ in mouse brain samples using total fluorescence

This example demonstrates the successful detection of aggregatory seeds of Aβ in samples obtained from brain extracts of CRND8[78] mice that are a model of Alzheimer's disease and littermate control mice. As in Example 2, the total population of beads was considered and the distribution of fluorescence intensities was measured.

This experiment used transgenic CRND8 mice that generate human Aβ₁₋₄₂ peptide in their brains. Wild type, littermate control mice were used as controls. Brain extracts were prepared using the protocol outlined below.
1. 200 mg mouse brain cortex was homogenized using a disposable plastic pestle in 50 µl of PBS + 0.05% (w/v) SDS.
2. The samples were then sonicated in an ice-cold water bath at maximum intensity for 8 min.
3. The samples were then centrifuged at 3000g for 1 min.
4. The supernatant was retained and the protein concentration of the extracts was determined using the BioRad protein assay (or nanodrop) and adjusted to 3 mg/ml by addition of PBS.
5. The samples were stored at -80°C.

The mouse brain samples were incubated with fluorescently labelled Aβ₁₋₄₂ monomers in microfluidic droplets. The total fluorescence of the droplets was measured using FACS analysis. As shown in Example 2, seeded aggregation of the fluorescently labelled peptide should be accelerated when compared to non-seeded aggregation, so it was expected that beads containing an aggregatory seed (from the non-labelled sample derived from CRND8 mice) should accumulate more fluorescent-labelled aggregate within a particular time and so have a higher fluorescence signal. Figure 14 compares the ratio of median fluorescence intensity for beads containing brain extracts divided by the median intensity of fluorescence in control beads that do not contain brain extract. Across a series of dilutions of the brain samples there was specific signal reporting the presence of Aβ₁₋₄₂ aggregatory seeds in CRND8, Alzheimer model mice (Figure 14, label AD) as compared to control brain extracts (label WT). The signal was seen for extract concentrations of 0.2 and 1 mg/ml.

### Example 12 - Detection of aggregatory seeds of Aβ in mouse serum samples using total fluorescence

This example demonstrates the successful detection of aggregatory seeds of Aβ in samples obtained from the serum of CRND8 [78] mice that are a model of Alzheimer's disease and littermate control mice. As in Example 2, the total population of beads was considered and the distribution of fluorescence intensities was measured.

This experiment used transgenic CRND8 mice that generate human Aβ₁₋₄₂ peptide in their brains. Wild type, littermate control mice were used as controls. Brain extracts were prepared using the protocol outlined below.
1. 300 µl of murine blood was allowed to clot in a 1.5 ml snap-top tube
2. The samples were then centrifuged at 3000g for 1 min.
3. The serum supernatant was retained and the protein concentration was determined using the BioRad protein assay (or nanodrop) and adjusted to 3 mg/ml by addition of PBS.
4. The samples were stored at -80°C.

The mouse serum samples at 100 µg/ml were incubated with fluorescently labelled Aβ₁₋₄₂ monomers in microfluidic droplets. The total fluorescence of the droplets was measured using FACS analysis. As shown in Example 2, seeded aggregation of the fluorescently labelled peptide should be accelerated when compared to non-seeded aggregation, so it was expected that beads containing an aggregatory seed (from the non-labelled sample derived from CRND8 mice) should accumulate more fluorescent-labelled aggregate within a particular time and so have a higher fluorescence signal. Figure 15 compares the fluorescence intensity for a population of >10,000 beads containing serum from diseases mice (CRND8 mouse, Alzheimer model serum, solid line) as compared to beads containing serum from littermate control mouse serum (dashed line). In the absence of brain extracts the fluorescence was at background levels, reporting spontaneous aggregation (dotted line).

The fluorescence intensity of the population of CRND8-derived beads was higher than for beads from control mouse serum. This indicates that aggregatory Aβ seeds can be detected in a peripheral tissue such as blood or serum at concentrations that are comparable to those in clinical specimens.

### REFERENCES

[1] Gupta et al., (2012) J. Biol. Chem., 287(13):9982-9989.
[2] Rosen et al., (2012) J. Neurochemistry, 120(5):660-6.
[3] Meyer-Luehmann et al., (2006) Science, 313:1781-4.
[4] Aguzzi et al., (2009) Neuron, 64:783-90.
[5] Berg et al., (2010) Nat. Protoc., 5:935-944.
[6] Anna et al., (2003) Appl. Phys. Lett., 82:364-366.
[7] Xu et al., (2005) Angew. Chem. Int. Ed. Engl., 44:724-728.
[8] Shah et al., (2008) Mater. Today, 11:18-27.
[9] Shah et al., (2008) Soft Matter, 4:2303-2309.
[10] Zhang et al., (2007) Macromol. Rapid Commun., 28:527-538.
[11] Oh et al., (2008) Prog. Polym. Sci., 33:448 - 477.
[12] Choi et al., (2007) Biomed. Microdevices, 9:855-862.
[13] Lin, Raffen, et al., (2001) Amyloid, 8(3):182-193.
[14] Meier, Kennedy-Darling, et al., (2009) Angew. Chem. Int. Ed. Engl., 48(8):1487-1489.
[15] Knowles, White, et al., (2011) Proc. Natl. Acad. Sci. USA, 108(36):14746-14751.
[16] Huebner, Sharma, et al., (2008) Lab Chip, 8(8):1244-1254.
[17] Schaerli and Hollfelder (2009) Mol. Biosyst., 5(12):1392-1404.
[18] Theberge. Courtois, et al., (2010) Angew. Chem. Int. Ed. Engl., 49(34):5846-5868.
[19] Casadevall i Solvas and deMello (2011) Chem. Commun. (Camb.), 47(7):1936-1942.
[20] Kintses, van Vliet, et al., (2010) Curr. Opin. Chem. Biol., 14(5):548-555.
[21] Link, Grasland-Mongrain, et al., (2006) Angew. Chem. Int. Ed. Engl., 45(16):2556-2560.
[22] Fidalgo, Whyte, et al., (2008) Angew. Chem. Int. Ed. Engl., 47(11):2042-2045.
[23] Baret, Miller, et al., (2009) Lab Chip 9(13):1850-1858.
[24] Niu, Gulati, et al., (2008) Lab Chip, 8(11):1837-1841.
[25] Mazutis, Baret, et al., (2009) Lab Chip, 9(18):2665-2672.
[26] Kiss, Ortoleva-Donnelly, et al., (2008) Anal. Chem., 80(23):8975-8981.
[27] Schaerli, Wootton, et al., (2009) Anal. Chem., 81(1):302-306.
[28] Courtois, Olguin, et al., (2008). Chembiochem 9(3):439-446.
[29] Huebner, Bratton, et al., (2009) Lab on a Chip, 9(5): 692-698.
[30] Huebner, Srisa-Art, et al., (2007) Chem. Commun. (Camb.), 12:1218-1220.
[31] Huebner, Olguin, et al., (2008) Anal. Chem., 80(10):3890-3896.
[32] Baret, Beck, et al., (2011) Chem. Biol., 17(5):528-536.
[33] Elder, Matthews, et al., (2006) Opt. Express., 14(12):5456-5467.
[34] Matthews, Elder, et al., (2007) Anal. Chem., 79(11):4101-4109.
[35] van Ham, Esposito, et al., (2010) J. Mol. Biol., 395(3):627-642.
[36] Chan, Kaminski, et al., (2011) Chemphyschem, 12(3):500-509.
[37] Kaminski, Schierle, Bertoncini, et al., (2011) Chemphyschem, 12(3):673-680.
[38] Xue Feng Wang et al., (1991) Applied Spectroscopy, 45(3):360-366.
[39] Becker et al. (2004) Microsc. Res. Tech., 63:58-66.
[40] Elder et al., (2006) Optics Express, 14:5456-5467.
[41] Chan et al., (2011) ChemPhysChem, 12(3):500-509.
[42] Kaminski et al.. (2011) J. Am. Chem. Soc., 133(33):12902-12905.
[43] Ahn et al., (2012) PLoS One, 7(11):e50192.
[44] Hamaguchi et al., (2012) Neuropathol., 123:31-7.
[45] Eisele et al., (2010) Science, 330:980-2.
[46] Clavaguera et al., (2009) Nat. Cell. Biol., 11:909-13.
[47] Collinge et al., (2007) Science, 318:930-6.
[48] Prusiner (1998) Proc. Natl. Acad. Sci. USA, 95:13363-83.
[49] Knowles et al., (2009) Science, 326:1533-7.
[50] Jarrett and Lansbury (1993) Cell, 73:1055-8.
[51] Polymenidou and Cleveland, (2012) J. Exp. Med., 209(5):889-93.
[52] Abate et al., (2009) Lab Chip, 9:2628-2631.
[73] Anna, Bontoux, et al. (2003) Applied Physics Letters, 82(3): 364-366.
[74] Holtze et al., (2008) Lab Chip, 8:1632.
[75] Chen et al., (2011) J. Am. Chem. Soc., 133:10368-10371.
[76] Crowther et al., (2005) Neuroscience, 132, 123-35.
[77] Crowther et al., (2006) Methods Enzymol., 412, 234-55.
[78] Chishti et α/., (2001) J. Biol. Chem. 276, 21562-70.
[79] Tokunaga et al., (2008) Nat. Methods, 5,159.
[80] Heilemann et al., (2010) Nat. Methods, 7, 717.
[81] Griess, Guiseley, et al., (1993) Biophysical journal, 65(1): 138-148.
[82] Griess, Moreno, et al., (1989) Biopolymers, 28(8): 1475-1484.
[83] Gillies, Wilhelm, et al., (2002) Nanotechnology, 13(5):587-591.
[84] Dobbie, King, et al., (2011) Cold Spring Harb. Protoc., 8: 899-909.
[85] Lesne, Koh, et al., (2006) Nature, 440(7082):352-357.
[86] Heilemann, Margeat, et al., (2005) Journal of the American Chemical Society, 127(11):3801-3806.
[87] Kaminski, Schierle, van de Linde, et al., (2011) J. Am. Chem. Soc., 133(33):12902-12905.
[88] Demuro, Smith, et al., (2011) J. Cell. Biol., 195(3):515-524.
[89] Frank et al., (2007) J. Microsc., 227 (Pt 3), 203-15.
[90] Yang, 2005
[91] Liu, 2004
[92] Murakami, et al., (2012) Hum. Mol. Genet., 21:1-9.
[93] Jahn et al., (2011) Neurosci. Methods, 197:186-9.
[94] Kohlhoff et al., (2011) Integr. Biol. (Camb.), 3:755-60.
[95] Jackson et al., (1998) Neuron,21:633-42.

## Claims

1. A method for detecting the presence of aggregatory seeds of a polypeptide in a sample, wherein an aggregatory seed is a soluble oligomer of the polypeptide, comprising:
(i) contacting the sample with monomers of the polypeptide, wherein the monomers of the polypeptide are labelled;
(ii) separating the sample into a plurality of microdroplets comprising a gel-forming agent in liquid form;
(iii) incubating the microdroplets under conditions suitable for aggregation of the polypeptide;
(iv) transforming the microdroplets into gel beads;
(v) determining the subsequent formation of aggregates of the polypeptide in the gel beads by detecting the label,
wherein the formation of aggregates of the polypeptide in a gel bead indicates the presence of one or more aggregatory seeds in the sample.

2. The method of claim 1 wherein said gel-forming agent is a polysaccharide, a polypeptide, a polyacrylamide, alginate, gelatine or agarose, and wherein said microdroplets are optionally transformed into gel beads by cooling.

3. The method of claim 1 or claim 2, wherein the monomers of the polypeptide are fluorescently labelled and the subsequent formation of aggregates of the polypeptide is determined by detecting a fluorescent signal.

4. The method of claim 3, wherein the detection of the fluorescent signal provides information regarding the structure of the polypeptide aggregates formed.

5. The method of claim 3 or claim 4, wherein the fluorescent signal is detected using fluorescence anisotropy imaging microscopy (FAIM), using a flow cytometer, using fluorescence lifetime microscopy, using direct stochastic optical reconstruction microscopy (dSTORM), using structured illumination microscopy, using FRET microscopy, or using circular dichroism.

6. The method of any one of the preceding claims, comprising a step of analysing the structure of polypeptide aggregates formed.

7. The method of any one of the preceding claims, wherein the microdroplets contain a reporter substance that produces a signal in the presence of aggregates of the polypeptide, and wherein the subsequent formation of aggregates of the polypeptide is determined by detecting the signal produced by the reporter substance.

8. The method of any one of the preceding claims, wherein the step of separating the sample into a plurality of microdroplets comprises emulsifying the sample into microdroplets.

9. The method of claim 8, wherein a water-in-oil emulsion is formed.

10. The method of any one of the preceding claims, wherein more than one technique or assay is used to detect or analyse the polypeptide aggregates.

11. The method of any one of the preceding claims, wherein the polypeptide is an Amyloid β peptide, such as Aβ₁₋₄₀ or Aβ₁₋₄₂, tau protein, prion protein, α-Synuclein, TDP-43, FUS, huntingtin, a serpin, transthyretin, Notch 3, glial fibrillary acidic protein, seipin, islet amyloid polypeptide (IAPP), pro-IAPP, apolipoprotein AI, All or AIV, gelsolin, β2 microglobulin, rhodopsin, keratin, tubulin, surfactant protein C, odontogenic ameloblast-associated protein, calcitonin, atrial natriuretic factor, serum amyloid A, medin, or lysozyme.

12. The method of any one of the preceding claims, wherein the method is for preparing reagents or products that are free from aggregatory seeds, or is for assessing the ability of a reagent to affect the formation of aggregatory seeds, and wherein an aggregatory seed is a soluble oligomer of the polypeptide.

## Patentansprüche

1. Verfahren zum Nachweisen des Vorhandenseins von Aggregationskeimen eines Polypeptids in einer Probe, wobei ein Aggregationskeim ein lösliches Oligomer des Polypeptids ist, umfassend:
(i) Inkontaktbringen der Probe mit Monomeren des Polypeptids, wobei die Monomere des Polypeptids markiert sind,
(ii) Auftrennen der Probe in eine Mehrzahl von Mikrotröpfchen, umfassend ein gelbildendes Mittel in flüssiger Form,
(iii) Inkubieren der Mikrotröpfchen unter Bedingungen, die zur Aggregation des Polypeptids geeignet sind, und
(iv) Umwandeln der Mikrotröpfchen in Gelkügelchen,
(v) Bestimmen der anschließenden Bildung von Aggregaten des Polypeptids in den Gelkügelchen durch Nachweisen der Markierung,
wobei die Bildung von Aggregaten des Polypeptids in einem Gelkügelchen auf das Vorhandensein eines oder mehrerer Aggregationskeime in der Probe hindeutet.

2. Verfahren nach Anspruch 1, wobei das gelbildende Mittel ein Polysaccharid, ein Polypeptid, ein Polyacrylamid, Alginat, Gelatine oder Agarose ist und wobei die Mikrotröpfchen gegebenenfalls durch Abkühlen in Gelkügelchen umgewandelt werden.

3. Verfahren nach Anspruch 1 oder Anspruch 2, wobei die Monomere des Polypeptids fluoreszenzmarkiert sind und die anschließende Bildung von Aggregaten des Polypeptids durch Nachweisen eines Fluoreszenzsignals bestimmt wird.

4. Verfahren nach Anspruch 3, wobei der Nachweis des Fluoreszenzsignals Informationen hinsichtlich der Struktur der gebildeten Polypeptidaggregate liefert.

5. Verfahren nach Anspruch 3 oder Anspruch 4, wobei das Fluoreszenzsignal unter Verwendung von Fluoreszenzanisotropie-Imaging-Mikroskopie (FAIM), unter Verwendung eines Durchflusszytometers, unter Verwendung von Fluoreszenzlebensdauer-Mikroskopie, unter Verwendung von direkter stochastischer optischer Rekonstruktionsmikroskopie (dSTORM), unter Verwendung von Mikroskopie mit strukturierter Beleuchtung, unter Verwendung von FRET-Mikroskopie oder unter Verwendung von Zirkulardichroismus nachgewiesen wird.

6. Verfahren nach einem der vorhergehenden Ansprüche, das einen Schritt des Analysierens der Struktur der gebildeten Polypeptidaggregate umfasst.

7. Verfahren nach einem der vorhergehenden Ansprüche, wobei die Mikrotröpfchen eine Reportersubstanz enthalten, die ein Signal in Gegenwart von Aggregaten des Polypeptids erzeugt, und wobei die anschließende Bildung von Aggregaten des Polypeptids durch Nachweisen des von der Reportersubstanz erzeugten Signals bestimmt wird.

8. Verfahren nach einem der vorhergehenden Ansprüche, wobei der Schritt des Auftrennens der Probe in eine Mehrzahl an Mikrotröpfchen das Emulgieren der Probe zu Mikrotröpfchen umfasst.

9. Verfahren nach Anspruch 8, wobei eine Wasser-in-Öl-Emulsion gebildet wird.

10. Verfahren nach einem der vorhergehenden Ansprüche, wobei mehr als eine Technik oder mehr als ein Assay zum Nachweisen oder Analysieren der Polypeptidaggregate verwendet wird.

11. Verfahren nach einem der vorhergehenden Ansprüche, wobei das Polypeptid ein Amyloid-β-Peptid, wie Aβ₁₋₄₀ oder Aβ₁₋₄₂, Tau-Protein, Prion-Protein, α-Synuclein, TDP-43, FUS, Huntingtin, ein Serpin, Transthyretin, Notch 3, saures Gliafaserprotein, Seipin, Insel-Amyloid-Polypeptid (IAPP), Pro-IAPP, Apolipoprotein AI, AII oder AIV, Gelsolin, β2-Mikroglobulin, Rhodopsin, Keratin, Tubulin, Surfactant-Protein C, odontogenes Ameloblast-assoziiertes Protein, Calcitonin, Atriumnatriuretischer Faktor, Serum-Amyloid A, Medin oder Lysozym ist.

12. Verfahren nach einem der vorhergehenden Ansprüche, wobei das Verfahren zur Herstellung von Reagenzien oder Produkten, die frei von Aggregationskeimen sind, oder zur Untersuchung der Fähigkeit eines Reagenzes, die Bildung von Aggregationskeimen zu beeinflussen, dient und wobei ein Aggregationskeim ein lösliches Oligomer des Polypeptids ist.

## Revendications

1. Méthode de détection de la présence de germes d'agrégation d'un polypeptide dans un échantillon, où un germe d'agrégation est un oligomère soluble du polypeptide, comprenant :
(i) la mise en contact de l'échantillon avec des monomères du polypeptide, où les monomères du polypeptide sont marqués ;
(ii) la séparation de l'échantillon en une pluralité de microgouttelettes comprenant un agent gélifiant sous forme liquide ;
(iii) l'incubation des microgouttelettes dans des conditions convenables pour l'agrégation du polypeptide ;
(iv) la transformation des microgouttelettes en billes de gel ;
(v) la détermination de la formation consécutive d'agrégats du polypeptide dans les billes de gel par la détection du marqueur ;
où la formation d'agrégats du polypeptide dans une bille de gel indique la présence d'un ou plusieurs germes d'agrégation dans l'échantillon.

2. Méthode selon la revendication 1, dans lequel ledit agent gélifiant est un polysaccharide, un polypeptide, un polyacrylamide, un alginate, de la gélatine ou de l'agarose, et où lesdites microgouttelettes sont éventuellement transformées en billes de gel par refroidissement.

3. Méthode selon la revendication 1 ou la revendication 2, dans laquelle les monomères du polypeptide sont marqués de manière fluorescente et la formation consécutive d'agrégats du polypeptide est déterminée par la détection d'un signal fluorescent.

4. Méthode selon la revendication 3, dans laquelle la détection du signal fluorescent fournit des informations concernant la structure des agrégats de polypeptide formés.

5. Méthode selon la revendication 3 ou la revendication 4, dans laquelle le signal fluorescent est détecté par imagerie d'anisotropie de fluorescence (FAIM), par cytométrie en flux, par microscopie de durée de vie de fluorescence, par microscopie de reconstruction optique stochastique directe (dSTORM), par microscopie à illumination structurée, par microscopie FRET, ou par dichroïsme circulaire.

6. Méthode selon l'une quelconque des revendications précédentes, comprenant une étape d'analyse de la structure des agrégats de polypeptide formés.

7. Méthode selon l'une quelconque des revendications précédentes, dans laquelle les microgouttelettes contiennent une substance rapporteuse qui produit un signal en présence d'agrégats du polypeptide, et où la formation consécutive d'agrégats du polypeptide est déterminée par la détection du signal produit par la substance rapporteuse.

8. Méthode selon l'une quelconque des revendications précédentes, dans laquelle l'étape de séparation de l'échantillon en une pluralité de microgouttelettes comprend l'émulsification de l'échantillon en microgouttelettes.

9. Méthode selon la revendication 8, dans laquelle une émulsion eau-dans-huile est formée.

10. Méthode selon l'une quelconque des revendications précédentes, dans laquelle plus d'une technique ou plus d'un dosage est utilisé(e) afin de détecter ou analyser les agrégats de polypeptide.

11. Méthode selon l'une quelconque des revendications précédentes, dans laquelle le polypeptide est un peptide β-amyloïde, tel que Aβ₁₋₄₀ ou Aβ₁₋₄₂, la protéine tau, une protéine prion, l'α-synucléine, TDP-43, FUS, l'huntingtine, une serpine, la transthyrétine, Notch 3, la protéine acide fibrillaire gliale, la séipine, le polypeptide amyloïde des îlots (IAPP), le pro-IAPP, l'apolipoprotéine AI, AII ou AIV, la gelsoline, la β2-microglobuline, la rhodopsine, la kératine, la tubuline, la protéine tensioactive C, la protéine associée aux améloblastes odontogéniques, la calcitonine, le facteur natriurétique auriculaire, la sérum amyloïde A, la médine, ou le lysozyme.

12. Méthode selon l'une quelconque des revendications précédentes, où la méthode est destinée à la préparation de réactifs ou de produits qui sont dépourvus de germes d'agrégation, ou est destinée à évaluer l'aptitude d'un réactif à affecter la formation de germes d'agrégation, et où un germe d'agrégation est un oligomère soluble du polypeptide.
